# EUROPEAN PATENT SPECIFICATION

(11) **EP 2 917 363 B1**
(45) Date of publication and mention of the grant of the patent: **09.01.2019**
(21) Application number: 13786285.0
(22) Date of filing: 06.11.2013
(51) Int. Cl.: C12Q 1/68

(54) **MEANS AND METHODS FOR IDENTIFYING RIBOSOME ASSOCIATED RNA MOLECULES**
MITTEL UND VERFAHREN ZUR IDENTIFIZIERUNG VON RIBOSOM-ASSOZIIERTEN RNA-MOLEKÜLEN
MOYENS ET PROCÉDÉS POUR IDENTIFIER DES MOLÉCULES D'ARN ASSOCIÉES AUX RIBOSOMES

(30) Priority: 06.11.2012 GB 201219976
(43) Date of publication of application: 16.09.2015
(73) Proprietor: Gerber, André, 5000 Aarau (CH); Nairz, Knud, 8006 Zürich (CH)
(72) Inventor: Gerber, André, 5000 Aarau (CH); Nairz, Knud, 8006 Zürich (CH)
(74) Representative: Ladendorf, Oliver
(86) International application number: PCT/EP2013/073179
(87) International publication number: WO 2014/072349

(56) References cited:
- WO-A2-2008/024499
- ALTHAUS H ET AL: "Cloning and sequencing of a cDNA expressing a ribosomal P0 peptide from Culicoides nubeculosus (Diptera).", VETERINARY IMMUNOLOGY AND IMMUNOPATHOLOGY MAY 2004, vol. 99, no. 1-2, May 2004 (2004-05), pages 99-111, XP002717202, ISSN: 0165-2427
- HALBEISEN R E ET AL: "Affinity purification of ribosomes to access the translatome", METHODS : A COMPANION TO METHODS IN ENZYMOLOGY, ACADEMIC PRESS INC., NEW YORK, NY, US, vol. 48, no. 3, 1 July 2009 (2009-07-01), pages 306-310, XP026284853, ISSN: 1046-2023, DOI: 10.1016/J.YMETH.2009.04.003 [retrieved on 2009-05-03]
- VENTOSO I ET AL: "Extensive translatome remodeling during ER stress response in mammalian cells", PLOS ONE 20120504 PUBLIC LIBRARY OF SCIENCE USA, vol. 7, no. 5, 4 May 2012 (2012-05-04), XP002717203, ISSN: 1932-6203
- THOMAS A ET AL: "A versatile method for cell-specific profiling of translated mrnas in Drosophila", PLOS ONE 20120706 PUBLIC LIBRARY OF SCIENCE USA, vol. 7, no. 7, 6 July 2012 (2012-07-06), XP055090877, ISSN: 1932-6203
- MUSTROPH ANGELIKA ET AL: "Profiling translatomes of discrete cell populations resolves altered cellular priorities during hypoxia in Arabidopsis", PROCEEDINGS OF THE NATIONAL ACADEMY OF SCIENCES OF THE UNITED STATES OF AMERICA, vol. 106, no. 44, November 2009 (2009-11), pages 18849-18854, XP002717204, ISSN: 0027-8424
- Francesca Gabanella ET AL: "SMN interacts with a novel family of hnRNP and spliceosomal proteins.", PLoS ONE, vol. 20, no. 9, 26 September 2007 (2007-09-26), page 5443, XP55335867, DOI: 10.1371/journal.pone.0000921
- DATABASE BIOSIS [Online] BIOSCIENCES INFORMATION SERVICE, PHILADELPHIA, PA, US; 1991, OGATA K ET AL: "ROLE OF 5S RRNA AS A POSITIVE EFFECTOR OF SOME AMINOACYL-TRNA SYNTHETASES IN MACROMOLECULAR COMPLEXES WITH SPECIFIC REFERENCE TO METHIONYL-TRNA SYNTHETASE", Database accession no. PREV199293075783

## Description

### FIELD OF THE INVENTION

The present invention relates to a method for identifying one or more RNA molecules, which are directly or indirectly associated with at least one ribosome, comprising: contacting a cellular extract with a molecule specifically binding to a ribosomal protein or a group of ribosomal proteins, wherein said specifically binding molecule is immobilized on a substrate, matrix or bead; and identifying one or more RNA molecules, which are directly or indirectly associated with at least one ribosome comprising said ribosomal protein or group of ribosomal proteins. The present invention further relates to a method for identifying one or more RNA molecules, which are differentially associated with at least one ribosome, comprising: contacting a cellular extract which is derived from cells grown under specific conditions, with a molecule specifically binding to a ribosomal protein or a group of ribosomal proteins; contacting a cellular extract derived from cells grown under normal conditions, with a molecule specifically binding to a ribosomal protein or a group of ribosomal proteins; and identifying RNA molecules present under the specific conditions, but not normal conditions, or vice versa. Furthermore, the present invention relates to a corresponding kit for detecting in a cellular extract one or more RNA molecules, which are directly or indirectly associated with at least one ribosome.

### BACKGROUND OF THE INVENTION

Organisms respond to environmental or physiological changes by altering the amounts and activities of specific proteins that are necessary for their adaptation and survival. Protein levels can be modulated by changing either the rate of synthesis or degradation, or the stability of messenger RNA, i.e. by modifying gene expression, or by changing the synthesis or stability of the protein itself. Gene expression can be regulated at diverse levels to achieve coordinate synthesis of the cell's macromolecular components. Besides transcriptional regulation, it has become increasingly evident that gene expression is controlled by a network of highly interconnected posttranscriptional regulatory factors, such as RNA-binding proteins and noncoding RNAs (Imig et al. 2012, Biomol. Concepts, 3: 395-486; Halbeisen et al., 2008, Cell Mol Life Sci, 65: 798-813; Keene, 2007, Nat Rev Genet, 8, 533-543). Consistently, the posttranscriptional regulation of protein synthesis plays essential roles for development, oncogenesis, and synaptic plasticity (Bilanges and Stokoe, 2007, Oncogene, 26, 5973-5990). Translation is thought to be mainly controlled at the initiation step where eukaryotic initiation factors (elFs) recruit the small ribosomal subunit (40S subunit) and scan the 5'-untranslated region (UTR) of the mRNA for the start codon. The initiation factors are then released, and the large ribosomal subunit (60S) joins the complex to form a fully assembled, translationally competent ribosome. Modification of initiation factors, such as phosphorylation of eIF2a, prevents formation of the initiation complex and thus globally represses translation initiation of most messages. Likewise, the availability of initiation factors, such as eIF4e, is controlled by 4E-binding proteins that inhibit association of the 40S subunit with the mRNA (Gebauer and Hentze, 2004, Nat Rev Mol Cell Biol, 5, 827-835). In yeast, the depletion of nutrients triggers such global repression within minutes, manifested by a gradual decrease of polysomes for most transcripts. The accumulating pool of mRNAs is typically incorporated into processing bodies (P-bodies) where they are degraded or kept translationally silent (Parker and Sheth, 2007, Mol Cell, 25, 635-646).

Besides global repression of translation, more specific modes of regulation can be observed for individual messages. For instance, translation of GCN4 is activated in response to amino acid deprivation - a condition that generally represses translation - by a mechanism that involves short upstream open reading frames (uORFs) (Hinnebusch, 1997, J Biol Chem, 272, 21661-21664). In addition, translation of specific messages can also be controlled by specific RNA binding proteins (RBPs) (Kong and Lasko, 2012, Nat. Rev. Genet. 13, 383-394; Gebauer and Hentze, 2004, Nat Rev Mol Cell Biol, 5, 827-835). Intriguingly, many regulatory RBPs interact with functionally related groups of mRNAs, so called posttranscriptional operons, suggesting highly coordinated control at all steps of posttranscriptional gene regulation (Gerber et al., 2004, PLoS Biol, 2, e79).

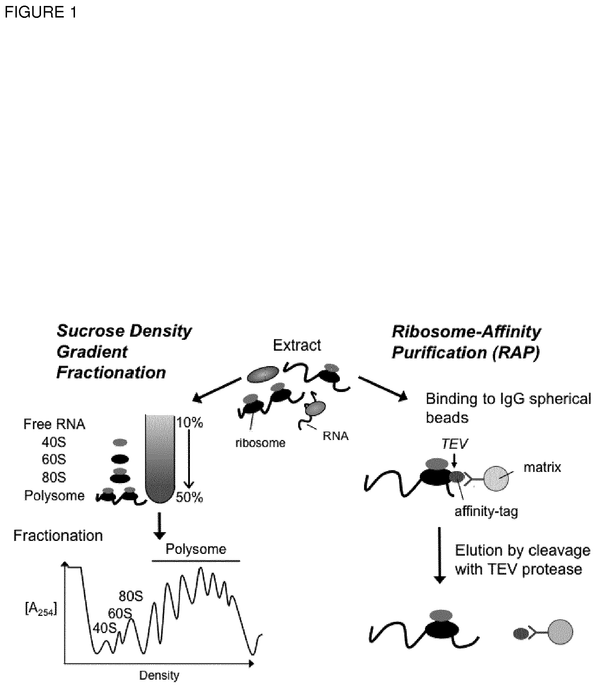

Several genome-scale studies have been undertaken to decipher the extent of translational regulation upon diverse stimuli in eukaryotes (reviewed in Halbeisen et al. 2008, Cell Mol Life Sci, 65: 798-813; Beilharz and Preiss, 2004, Brief Funct Genomic Proteomic, 3, 103-111). Classical sucrose density fractionation has been commonly applied to separate free RNA and monosomes from polysomes, followed by a systematic analysis of the relative distribution of mRNAs in these two fractions using DNA microarrays (see, for instance, Fig. 1). In most cases, the global response on transcript levels was quantified in parallel to disclose the relation between nascent transcription/RNA turnover and translation (Kuhn et al., 2001, Mol Cell Biol, 21, 916-927).

For several stress conditions such as amino acid deprivation, high salinity, and heat shock, the imposed remodeling of translation often reflects a magnification of the changes of transcript levels. This effect has been termed "potentiation" and may in part be explained by the deposition of regulatory proteins on certain mRNAs during transcription (Preiss et al., 2003, Nat Struct Biol, 10, 1039-1047). However, under other stress conditions, such as butanol or peroxide addition, these coordinate reactions could not be observed (Shenton et al., 2006, J Biol Chem, 281, 29011-29021). Clearly, details about the extent and regulation of potentiation remain to be resolved. Nevertheless, the specific sets of mRNAs that underwent treatment specific regulation shared some common functional theme that can be attributed to a logical response of the cell to its altered physiological circumstances. The above-mentioned studies exposed cells to severe stress conditions, triggering global repression of translation and cell growth. However, it is well established that under these conditions, yeast cells respond in a stress-specific manner but also by a common program that changes the steady-state level of hundreds of messages in a stereotypic way, commonly referred to as the environmental stress response or ESR (Gasch et al, 2000, Mol Biol Cell, 11, 4241-4257).

In the yeast Saccharomyces cerevisiae, ESR includes approximately 900 genes; 600 genes are commonly repressed and mainly encode proteins for growth-related processes including ribosomal proteins, whereas 300 genes are up-regulated and refer to proteins acting in carbohydrate metabolism, intracellular signaling, or stress, such as chaperones and DNA damage repair enzymes (Gasch et al, 2000, Mol Biol Cell, 11, 4241-4257). In contrast, the number and nature of messages that undergo translational regulation under rather mild stress conditions, i.e., stimuli that affect cellular homeostasis without triggering ESR, and thus reflecting adaptive responses that may occur very frequently in a cell has not been analyzed in great detail.

In Halbeisen et al., 2009, Methods, 48(3), 306-310 a ribosome affinity purification (RAP) method is described which allows rapid purification of ribosomes and associated messages from the yeast Saccharomyces cerevisiae. The method relies on the expression of protein A tagged versions of the ribosomal protein Rpl16, which is used to efficiently recover endogenously formed ribosomes and polysomes from cellular extracts with IgG-coupled spherical microbeads. Ventoso et al., 2012, PLOS one, 7, 5, e35915, 1-12 discloses extensive translatome remodeling during ER stress response in mammalian cells. Described are the translatomes of two mammalian cell lines, NIH3T3 and Jurkat, by scoring the relative polysome association of about 10,000 mRNA under normal and ER stress conditions. It was found that translation efficiencies of mRNA correlated poorly with transcript abundance, although a general tendency was observed so that the highest translation efficiencies were found in abundant mRNA.

A systematical comparison of global transcript levels (transcriptome) with global alterations in the levels of ribosome association of transcripts (translatome) in response to different stresses in yeast, which was methodologically based on the purification of affinity-tagged ribosomes, was carried out in Halbeisen and Gerber, PLOS, 7(5), 2009, e1000105 to determine how significant the discrepancy between transcript and protein levels can be (see Fig. 1). It was found that changes in the transcriptome correlate well with those in the translatome after application of harsh stresses that arrest cell growth. However, this correlation was generally lost in scenarios, which do not affect cell growth. In these cases, remodeling of gene expression was mainly executed at the translational level by modulating mRNA association with ribosomes. Yet this approach is confined to affinity-tagged ribosomes and accordingly does not allow identifying alteration in the translatome of non-modified subjects. There is, thus, a need for an improved methodology for identifying one or more RNA molecules, which are directly or indirectly associated with at least one ribosome, representing the translatome, in particular in organisms which are genetically not easily amenable.

### OBJECTS AND SUMMARY OF THE INVENTION

The present invention addresses this need and provides a method for identifying one or more RNA molecules, which are directly or indirectly associated with at least one ribosome, comprising: (a) contacting a cellular extract with a molecule specifically binding to a ribosomal protein or a group of ribosomal proteins, wherein said specifically binding molecule is immobilized on a substrate, matrix or bead; and (b) identifying one or more RNA molecules, which are directly or indirectly associated with at least one ribosome comprising said ribosomal protein or group of ribosomal proteins.

The inventors found that the purification of RNA molecules which are directly or indirectly associated with ribosomes can be achieved by using molecules which specifically bind to a ribosomal protein or a group of ribosomal proteins. The use of such binding molecules makes it possible to isolate ribosomes and nucleic acids bound thereon without the need of molecularly modifying ribosomal proteins, in particular without being forced to reengineer target cells by introducing coding sequences for affinity tags into the genome. The surprising finding that the employment of molecules specifically binding ribosomal proteins or groups of ribosomal proteins allows to effectively isolate and purify ribosomes and RNAs associated therewith now offers the possibility of extending the analysis of translatome regulations to non-modified or native cells. Thus, cellular extracts derived from different organisms, tissues and cells raised at differential conditions can be used and thus facilitate the identification of differentially regulated proteins in a huge number of contexts. Moreover, the technology has the potential to reveal non-coding RNAs that are differentially loaded to ribosomes. These proteins - in fact detected by their nascent transcripts - and non-coding ribosome-associated RNAs have a high diagnostic potential.

In a specific embodiment of the present invention said cellular or tissue extract is derived from cells or tissues grown under or obtained from conditions selected from the group of: metabolic stress conditions, specific nutrient and/or supply conditions, conditions involving the absence or presence of one or more growth factors, differentiation factors, or receptor ligands, conditions involving the absence or presence of pharmaceutically active compounds, potentially pharmaceutically active compounds or small molecules, toxic or potentially toxic conditions, pathological or potentially pathological conditions; and normal, healthy or optimal conditions.

The present invention also relates in a further specific embodiment to a method as defined herein above, comprising the identification of one or more RNA molecules associated with at least one ribosome under two or more of conditions selected from the group of: metabolic stress conditions, specific nutrient and/or supply conditions, conditions involving the absence or presence of one or more growth factors, differentiation factors, or receptor ligands, conditions involving the absence or presence of pharmaceutically active compounds, potentially pharmaceutically active compounds or small molecules, toxic or potentially toxic conditions and/or pathological or potentially pathological conditions and normal, healthy or optimal conditions, preferably including a comparison of said identified RNA molecules obtained under two or more of said conditions.

In a further aspect the present invention relates to a method for identifying one or more RNA molecules, which are differentially associated with at least one ribosome, comprising:
(a) contacting a cellular extract, wherein said cellular extract is derived from cells grown under or obtained from conditions selected from the group of: metabolic stress conditions, different nutrient and/or supply conditions, conditions involving the absence or presence of one or more growth factors, differentiation factors, receptor ligands, conditions involving the absence or presence of pharmaceutically active compounds, potentially pharmaceutically active compounds or small molecules, toxic or potentially toxic conditions and/or pathological or potentially pathological conditions, with a molecule specifically binding to a ribosomal protein or a group of ribosomal proteins, wherein said specifically binding molecule is immobilized on a substrate, matrix or bead;
(b) contacting a cellular extract, wherein said cellular extract is derived from cells grown under normal, healthy and/or optimal conditions, with a molecule specifically binding to a ribosomal protein or a group of ribosomal proteins, wherein said specifically binding molecule is immobilized on a substrate, matrix or bead; and
(c) identifying one or more RNA molecules being directly or indirectly associated with at least one ribosome comprising said ribosomal protein or group of ribosomal proteins which are present under conditions of step (a), but not under conditions of step (b), or one or more RNA molecules which are present under conditions of step (b), but not under conditions of step (a).

In a preferred embodiment of the present invention the RNA molecule as mentioned above is an mRNA or non-coding RNA molecule, which is directly associated with at least one ribosome, or a non-coding RNA molecule, which is indirectly associated with at least one ribosome.

In a further preferred embodiment essentially all or all mRNA or non-coding RNA molecules which are directly associated with at least one ribosome are identified.

In yet another preferred embodiment essentially all or all non-coding RNA molecules, which are indirectly associated with at least one ribosome are identified.

In a further preferred embodiment said indirect association with at least one ribosome comprises a direct association of said non-coding RNA molecule with said mRNA molecule, wherein said mRNA molecule is directly associated with at least one ribosome.

According to further preferred embodiments of the invention, said non-coding RNA molecule is a microRNA molecule, long ncRNA molecule, short ncRNA molecule, siRNA molecule, antisense RNA molecule, or a regulatory RNA molecule.

In a further embodiment of the present invention said identification of one or more RNA molecules comprises an amplification of the RNA molecule to a nucleic acid molecule. The present invention further envisages that said amplified nucleic acid molecule is additionally sequenced. It is preferred that the sequencing is performed as high throughput sequencing, microarray based hybridization sequencing or library based sequencing.

In a further preferred embodiment said identification of one or more RNA molecules comprises a determination of the sequence of the RNA molecule. It is particularly preferred that the determination is performed by sequencing of reverse transcribed complementary DNA (cDNA) or direct RNA sequencing.

In another preferred embodiment of the present invention said specifically binding molecule is an antibody, intrabody, antibody substructure, or modified antibody, or a fragment thereof. Said antibody, intrabody, antibody substructure, or modified antibody, or fragment thereof may specifically bind to a ribosomal protein located at the surface of a ribosome; and/or to a ribosomal protein which is or becomes temporary or conditionally accessible to the antibody, intrabody, antibody substructure, or modified antibody, or fragment thereof.

In a further embodiment of the present invention, said specifically binding molecule is a non-immunoglobulin protein. Preferably, said non-immunoglobulin protein binds to a ribosomal protein or a group of ribosomal proteins located at the surface of a ribosome; and/or to a ribosomal protein or a group of ribosomal proteins, which is/are or become(s) temporary or conditionally accessible to the non-immunoglobulin protein.

In a preferred embodiment, said non-immunoglobulin protein is selected from the group of: designed ankyrin-repeat protein (DARPin), affibody molecule, adnectin, anticalin, affilin, avimer, knottin, fynomer, phylomer, artificial antibody mimic and kunitz domain peptide.

In a further preferred embodiment said artificial antibody mimic is a molecularly imprinted polymer (MIP). Particularly preferred molecularly imprinted polymers (MIPs) are acrylamide based polymers, polyethylene based polymers, acrylic acid based polymers, or derivatives thereof. Preferred derivatives are methacrylic acid based polymers and methyl methacrylate based polymers. Also envisaged is the use of any combination or mixture of the above mentioned polymers.

In a further embodiment of the invention, said specifically binding molecule is an aptamer. The present invention envisages that the aptamer is a nucleic acid aptamer, or a peptide aptamer. It is preferred that said aptamer binds to a ribosomal protein located at the surface of a ribosome and/or to a ribosomal protein which is or becomes accessible to the aptamer.

In a further preferred embodiment of the present invention said specifically binding molecule is ribosome binding protein p34 (LRRC59), or ER membrane translocator SEC61alpha, or a fragment thereof.

It is further preferred that said ribosomal protein is a eukaryotic ribosomal protein or a prokaryotic or archaea homolog thereof.

In a particularly preferred embodiment said eukaryotic ribosomal protein is a mammalian ribosomal protein. More preferably, said mammalian ribosomal protein is a human ribosomal protein.

In another embodiment of the present invention, said mammalian ribosomal protein is a human mitochondrial ribosomal protein.

It is further envisaged by the present invention that said ribosomal protein is at least one ribosomal protein, which is located at the 60S solvent-exposed side of a ribosome.

In a preferred embodiment of the present invention said ribosomal protein is at least one ribosomal protein selected from the group of: Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A,Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, and Homo sapiens ribosomal protein L38, or a homologue thereof of a eukaryotic, prokaryotic or archaea species.

It is particularly preferred that said ribosomal protein is Homo sapiens ribosomal protein L13A, Homo sapiens ribosomal protein L18, Homo sapiens ribosomal protein L22 or Homo sapiens ribosomal protein L26.

In another embodiment of the present invention said cellular extract is an extract derived from a prokaryotic cell, an archaea or archaebacterial cell, a eukaryotic cell, a mitochondrium, or the cytoplasm of a eukaryotic cell. Said eukaryotic cell may preferably be a mammalian cell. More preferably, said mammalian cell may be a human cell.

In a further embodiment said mitochondrium is a mitochondrium of a mammalian cell, preferably a mitochondrium of a human cell.

In a preferred embodiment of the present invention, said cellular extract is a tissue extract derived from a eukaryotic organism.

In yet another preferred embodiment, said tissue extract is a mammalian tissue extract, more preferably a human tissue extract.

In a preferred embodiment of the invention, said RNA molecule is an mRNA or non-coding RNA molecule which is directly associated with at least one ribosome or a non-coding RNA molecule, which is indirectly associated with at least one ribosome.

In yet another aspect the present invention relates to a kit for identifying in a cellular extract as defined herein above one or more RNA molecules as defined herein above, which are directly or indirectly associated with at least one ribosome, comprising:
(i) a molecule which is specifically binding to a ribosomal protein or a group of ribosomal proteins as defined herein above, wherein said molecule is immobilized on a substrate, matrix or bead; and
(ii) means for identifying one or more RNA molecules, which are directly or indirectly associated with at least one ribosome comprising said ribosomal protein or group of ribosomal proteins.

In a preferred embodiment of the present invention said means for identifying one or more RNA molecules, which are directly or indirectly associated with at least one ribosome comprising said ribosomal protein or group of ribosomal proteins, comprises means for amplifying the RNA molecule to a nucleic acid molecule.

In another preferred embodiment said kit further comprises means for sequencing said amplified nucleic acid molecule.

In yet another preferred embodiment of the present invention, said means for identifying one or more RNA molecules, which are directly or indirectly associated with at least one ribosome comprising said ribosomal protein or group of ribosomal proteins, comprises means for directly sequencing the RNA molecule.

In a further specific embodiment of the present invention said kit additionally comprises an RNase activity.

### BRIEF DESCRIPTION OF THE FIGURES

**Fig. 1** shows a comparison of methods hitherto used in the field of translatomics. On the left hand side the employment of sucrose density gradients is depicted, leading to the provision of density fractioned ribosome associated entities. On the right hand side the use of ribosome-affinity purification (RAP) is depicted, which essentially relies on the presence of an affinity tag in a ribosomal protein.

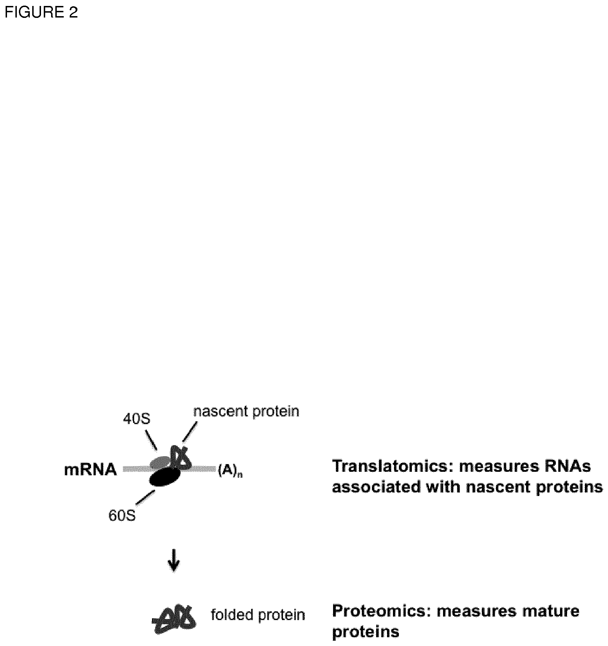

**Fig. 2** depicts the difference between proteomics, i.e. the measurement of expressed proteins in a cell, and translatomics, i.e. the measurement of actively transcribed and ribosome associated RNA molecules in a cell.

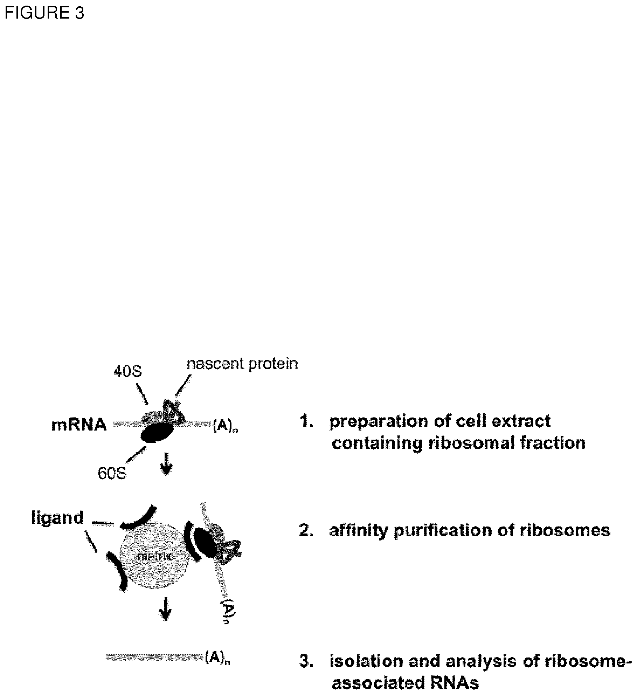

**Fig. 3** gives a schematic overview over the principle underlying the present invention. Step 1 shows the provision of cellular extracts comprising ribosomal structures comprising associated nucleic acids. Step 2 depicts the affinity based purification of these ribosomal structures via interaction with a molecule specifically binding to the ribosome. Step 3 depicts the subsequent isolation and analysis of RNA molecules.

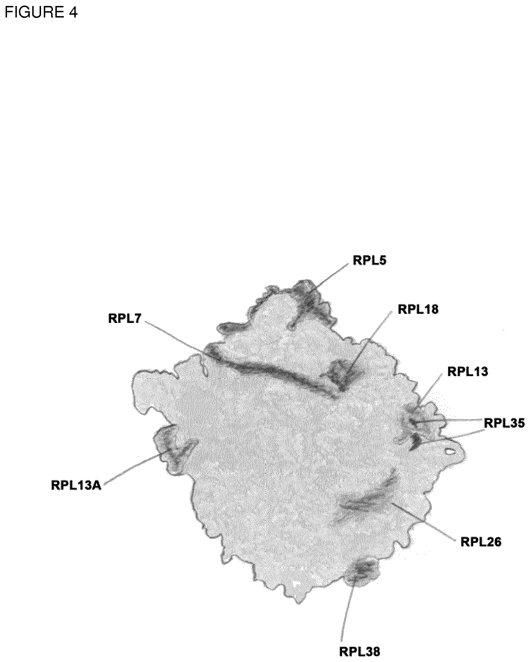

**Fig. 4** shows the three-dimensional structure of the solvent accessible site of the 60S ribosomal subunit of a Tetrahymena thermophila eukaryotic ribosome, which shows high structural homology to mammalian ribosomes. Shaded in dark grey are epitopes of ribosomal proteins that are regarded as most promising and accessible structures to interact with molecules with specific binding activity.

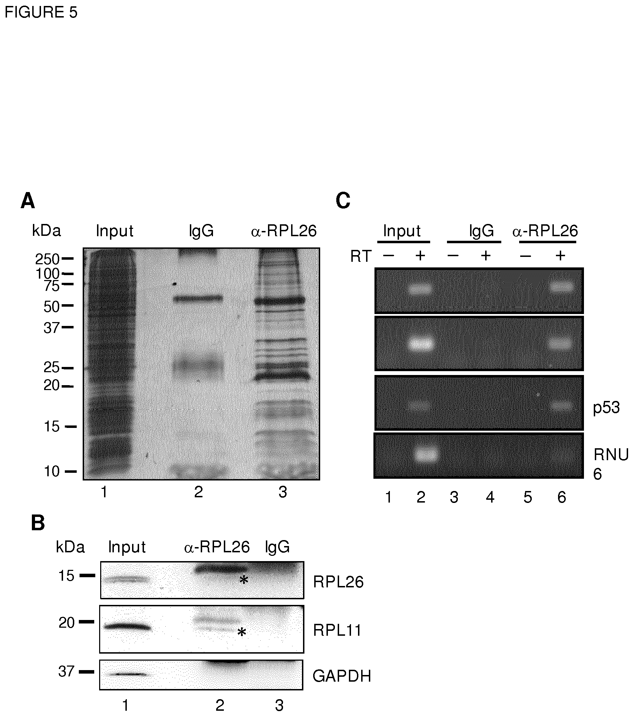

**Fig. 5** shows the results of an immunoprecipitation (IP) of ribosomal proteins and associated mRNA from HEK293 cell lysates using rabbit-anti-RPL26 monoclonal antibodies. In **Fig. 5** **A** silver-stained polyacrylamide gels were used to visualize proteins or RNAs. Lane 1 (input) represents a sample from the extract. The input extract was then either incubated with IgG coupled beads (lane 2) or anti-RPL26 coupled beads (lane 3). The beads were washed with physiological buffers and bound proteins/RNAs were eluted and loaded on the gel. Application of anti-RPL26 (lane 3) antibodies shows multiple bands representing proteins/RNAs enriched in the eluates, which are absent in negative control eluates with IgG (lane 2). In **Fig. 5** **B** results of an immunoblot analysis of extract (Input, lane 1), and eluates from anti-RPL26 IP (lane 2) and control IPs (IgG, lane 3) are shown. RPL26 (upper part), RPL11 (middle; another ribosomal protein of the large subunit) are identified in the anti-RPL26 eluates but not in the control (* demarks band corresponding to RPLs, an upper strong band represents cross-reaction with IgG chains). GAPDH (lower part) is not expected to interact with ribosomes and serves as a control. **Fig. 5** **C** provides results of reverse transcription (RT)-PCR analysis of RNA isolated from RPL26 and control IPs. Products were run on an agarose gel and stained with ethidium bromide. Messenger RNAs for GAPDH, NFkB and p53 are specifically enriched in anti-RPL26 IP samples (lane 6) compared to control IgG samples (lane 4). RNU6 refers to a small nuclear RNA not expected to be associated with ribosomes. Lanes 1, 3, 5 represent assays without addition of RT, which is a negative control to test for potential contamination with genomic DNA or fragments thereof. Input (lanes 1, 2) relates to total RNA isolated from the extract (positive control).

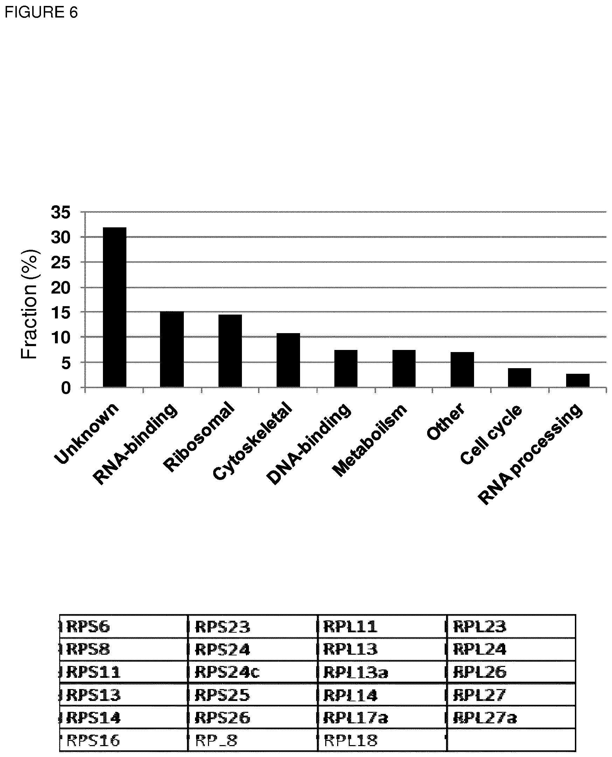

**Fig. 6** illustrates the results of an MS analysis of a representative sample shown in Fig. 5A.

### DETAILED DESCRIPTION OF THE INVENTION

The present invention relates to a method for identifying one or more RNA molecules, which are directly or indirectly associated with at least one ribosome, comprising: (a) contacting a cellular extract with a molecule specifically binding to a ribosomal protein or a group of ribosomal proteins, wherein said specifically binding molecule is immobilized on a substrate, matrix or bead; and (b) identifying one or more RNA molecules, which are directly or indirectly associated with at least one ribosome comprising said ribosomal protein or group of ribosomal proteins.

Although the present invention will be described with respect to particular embodiments, this description is not to be construed in a limiting sense.

Before describing in detail exemplary embodiments of the present invention, definitions important for understanding the present invention are given.

It must be noted that as used herein and in the appended claims, the singular forms "a", "an", and "the", include plural referents unless the context clearly indicates otherwise. Thus, for example, reference to "a polypeptide" or "a ribosome" includes one or more of such polypeptides or ribosomes, and reference to "the method" includes reference to equivalent steps and methods known to those of ordinary skill in the art that could be modified or substituted for the methods described herein.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of'. If hereinafter a group is defined to comprise at least a certain number of embodiments, this is meant to also encompass a group, which preferably consists of these embodiments only.

Furthermore, the terms "first", "second", "third" or "(a)", "(b)", "(c)", "(d)" or "(i)", "(ii)", "(iii)", "(iv)", "(v)", "(vi)", "(vii)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" or "(i)", "(ii)", "(iii)", "(iv)", "(v)", "(vi)", "(vii)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application or claims as set forth herein above or below.

It is to be understood that this invention is not limited to the particular methodology, protocols, proteins, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a method for identifying one or more RNA molecules, which are directly or indirectly associated with at least one ribosome, comprising: (a) contacting a cellular extract with a molecule specifically binding to a ribosomal protein or a group of ribosomal proteins, wherein said specifically binding molecule is immobilized on a substrate, matrix or bead; and (b) identifying one or more RNA molecules, which are directly or indirectly associated with at least one ribosome comprising said ribosomal protein or group of ribosomal proteins.

The term "RNA molecule" as used herein refers to a nucleic acid, in particular a ribonucleic acid. The RNA molecule may further, in principle, be an mRNA or a non-coding RNA molecule. The mRNA molecule is typically translated by the ribosome and thus directly associated with at least one ribosome. It may also be associated with more than one ribosome, e.g. in the case of polysomes. The term "non-coding RNA molecule" as used herein refers to a non-mRNA molecule, i.e. to a molecule which is not translated or translatable by a ribosome. The non-coding molecule is, in preferred embodiments of the present invention, a microRNA molecule, a long ncRNA molecule, a short ncRNA molecule, a siRNA molecule, an antisense RNA molecule, or a regulatory RNA molecule. Further details are known to the skilled person or can be derived from suitable literature sources such as Esteller, 2011, Nat. Rev. Genet. 12: 861-874.

The term "microRNA" as used herein means a short single-stranded RNA with about 19 to 22 nucleotides, with sequence complementarity to mRNAs. Typically, its most 5' nucleotides may be complementary to mRNAs. miRNAs may confer repressive activity by binding to target mRNAs and by inhibiting protein translation (see also Krol et al., 2010 Nat. Rev. Genet. 9: 597-5610). The term "long ncRNA molecule" as used herein means an RNA with a length of about 200 nucleotides or longer and without an open reading frame of about 50 nucleotides or longer. Long non-coding RNAs may typically not be translated into proteins. In certain, specific cases, long ncRNAs may be translated into polypeptides. Examples of ncRNAs which are translated into polypeptides are short, polycistronic ribosome-associated coding RNAs (sprcRNAs) as described in Ingolia et al. 2011, Cell, 7: 789-802. The term "short ncRNA" as used herein means an RNA molecule with about 20 to 200 nucleotides that is not translated into a protein and which performs a regulatory activity. The term "siRNA molecule" as used herein means a short double-stranded RNA with about 19 to 22 nucleotides each. The siRNA typically comprises a 5' phosphate group, a 3' hydroxyl group, and may further comprise 2 nucleotides overhang. The siRNA molecule may show sequence complementarity to mRNAs. siRNAs typically confer repressive activity by the RNA interference pathway, which may lead to the degradation of RNAs with sequence homologies. The term "antisense RNA molecule" as used herein means a single-stranded RNA with sequence complementarity to mRNAs and which inhibit translation by base-pairing. The term "regulatory RNA molecule" as used herein means an molecule that is not translated into a protein, but performs a regulatory function by either directly or indirectly affecting translation, decay and localization of targets mRNA molecules or by exerting a function in the nucleus through associating with genomic DNA that can have impact on chromatin structure and modification. Further details may be derived from suitable literature sources such as Esteller M 2011, Nat. Rev. Genet. 12: 861-874.

mRNA molecules and also non-coding RNA molecules may be directly associated with at least one ribosome. For instance, non-coding RNA molecules may directly bind to ribosomal proteins or other components of the ribosomal complex. Non-coding RNA molecules may further be indirectly associated with at least one ribosome, e.g. by not being bound to the ribosome itself, but by being bound to an mRNA molecule, which in turn is bound to the ribosome.

The term "directly associated with at least one ribosome" as used herein thus means that the RNA molecule is in physical contact with one ribosome, or with more than one ribosome. Such physical contact may involve non-covalent bindings which include interactions such as hydrogen bonding, electrostatic interactions, Van der Waals interactions, and hydrophobic packing.

The term "indirectly associated with at least one ribosome" as used herein refers to an association of the RNA molecule to one or more ribosomes, which is not provided by a physical contact between said RNA molecule and said ribosome(s), but is provided by a linking or mediating entity. Such an entity can be a further molecule such as an RNA molecule. It can further be a protein or protein fragment which is positioned between a ribosome and said RNA molecule.

The term "identifying an RNA molecule" as used herein refers to one or more activities, which result in the elucidation of the nature and/or sequence of the RNA molecule. The identification may comprise isolation activities, amplification activities, sequencing activities, comparison steps with database or literature sources. In certain cases identification may also include isolation steps, which do not directly lead to the determination of the sequence of an RNA molecule, but allow for such a determination in subsequent or future steps. Accordingly, RNA molecules as defined herein, or derived nucleic acids, may be deposited, cloned and/or otherwise safeguarded for molecular analyses, e.g. sequence determination, in subsequent steps or in the future.

In a specific embodiment, the identification of one or more RNA molecules comprises, as one step, the amplification of the RNA molecule into a nucleic acid molecule. The term "nucleic acid" as used herein refers to DNA, RNA or a derivative thereof. The nucleic acid is either single stranded or double stranded and may be comprised of DNA-RNA hybrids as well. Derivatives of the nucleic acid may include modified nucleic acids, e.g. nucleic acids comprising additional or modified chemical groups, additional charges in the nucleic acid, entities conveying additional or differing additional or differing electrostatic interaction, additional or differing hydrogen bonding, or differing or additional functionality. In the context of an amplified RNA molecule, it is preferred that the nucleic acid is a DNA molecule, or a modified DNA molecule. Envisaged examples of a modification of the nucleic acid are phosphodiester group modifications, 2'-position sugar modifications, 5'-position pyrimidine modifications, purine modifications backbone modifications, methylations, unusual base-pairing combinations such as the isobases isocytidine and isoguanidine. Further modifications can also include 3' and 5' modifications such as capping moieties. Also envisaged are modifications based on 2'-deoxy nucleic acid linkers.

Also envisaged is the modification of nucleic acids via labeling. Such a labeling activity may be based on conjugation reactions using various chemistries, e.g. aldehyde, carboxylic acids, or amine reactions. Examples of labels that can be conjugated to a nucleic acid include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). Further labels which may be used within the context of the present invention include 6-FAM, HEX, TET, ROX, Cy3, Cy5, Texas Red or Rhodamine, TAMRA, Dabcyl, Black Hole Quencher, BHQ-1 or BHQ-2. Target molecules may also be labeled with radioisotopes e.g. ³H, ¹⁴C, ³²P, ³³P, ³⁵S, ¹²⁵I, ¹¹C, ¹³N, 15O, ¹⁸F, ⁶⁴Cu, ⁶²Cu, ¹²⁴I, ⁷⁶Br, ⁸²Rb, ⁶⁸Ga or ¹⁸F.

The term "amplification" as used herein means that the RNA molecule is provided in or transformed into an amplificable state and subsequently exposed to a process including at least one round of extension, replication, or transcription in order to increase the number of nucleic acid molecule copies. The present invention envisages any suitable method of amplification of nucleic acids, in particular of RNA molecules. Rounds of extension and replication may preferably be implemented in the form of a polymerase chain reaction protocol (PCR). In order to be provided in an amplifiable state the RNA molecule is preferably first reverse transcribed into a DNA (cDNA) molecule. On the basis of such a cDNA molecule further amplification steps may be carried out. For the reverse transcription a reverse transcriptase may be used, e.g. AMV or M-MLV reverse transcriptases.

In addition, primers which bind to polyA tails may be employed. In case of non-coding RNAs a ligation protocol for terminal extension of the molecule may be used. Examples of PCR methods or PCR based methods for amplification of nucleic acids, which are envisaged by the present invention, include basic PCR, hot-start PCR, long PCR, quantitative endpoint PCR, quantitative real-time PCR, rapid amplified polymorphic DNA analysis, rapid amplification of cDNA ends, differential display PCR, in situ PCR, and high fidelity PCR. Further details would be known to the skilled person or can be derived from suitable literature sources such as: Saiki et al., 1985, Science, 230: 1350-1354; Nanashima et al., 2008, J. Biol. Chem., 283: 16868-16875; Carothers et al., 1989, Biotechniques, 7: 494-9 1989; Bames, 1994, Proc. Natl. Acad. Sci. USA, 91: 2216-20; Gaudette and Crain, 1991, Nucl. Acids Res. 19: 1879-1884; Lee et al., 1993, Nucl. Acids Res., 21: 3761-3766; McClelland and Welsh, 1994, PCR Methods Appl., 4:S59-65; Troutt et al., 1992, Proc. Natl. Acad. Sci. USA, 89: 9823- 9825, Liang and Pardee, 1992, Science, 257: 967-71; Haase et al., 1990, Proc. Natl. Acad. Sci. USA 87: 4971-4975, and Cline et al., Nucl. Acids Res. 1996, 24: 3546- 3551. Further envisaged amplification methods include isothermal amplification processes such as NASBA or Rolling Circle amplification (RCA), which are known to the person skilled in the art.

Amplification methods may further include amplifications on beads. Examples of such methods include emulsion methods. In order to use emulsion based amplification techniques with a single template per emulsion bubble, a single primer is typically attached to a bead, and a single primer is in solution, thereby amplifying the templates such that one end of the duplex is attached to the bead. Subsequently, the hybridized strand can be removed by denaturing the duplex. This leaves the immobilized single strand on the bead. Subsequently, the single stranded templates can be captured, for example, onto a surface via primers complementary to the templates. Further details may be derived from Shendure and Hanlee, 2008, Nat. Biotech, 26:1135-1145.

Also envisaged are surface-using amplification forms such as bridge amplification to form nucleic acid clusters. The term "bridge amplification" refers to a solid phase replication method in which primers are bound to a solid phase, e.g., flow cell, microarray, and the like. The term "bridge" in this context refers to the fact that during the annealing step, the extension product from one bound primer forms a bridge to the other bound primer. All amplified products are covalently bound to the surface, and can be detected and quantified without electrophoresis. Sequencing by synthesis methods may be employed with any appropriate amplification method, including, for example, PCR.

In specific embodiments, the amplified nucleic acid is sequenced. For such a sequencing approach any suitable sequencing method can be applied. For example, the sequence may be obtained by using Massively Parallel Signature Sequencing (MPSS).

An example of an envisaged sequence method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing typically uses luciferase to generate light for detection of the individual nucleotides added to the nascent DNA, and the combined data are used to generate sequence read-outs.

Another envisaged example is Illumina or Solexa sequencing i.e. polymerase-based sequence-by-synthesis, e.g. by using the Illumina Genome Analyzer technology. This technique uses reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle.

Yet another example is the use of Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing.

Another example of a sequencing method envisaged by the present invention is sequencing by nanopores. In this method the target nucleic acid or nucleotides released from the target nucleic acid pass through a nanopore. The nanopore can be, for example, a synthetic pore or biological membrane protein, such as a hemolysin. As the target nucleic acid or nucleotides pass through the nanopore, each base-pair (or base) can be identified by measuring fluctuations in the electrical conductance of the pore. Further information may be derived, for example, from Soni, G.V. and Meller, 2007, Clin. Chem. 53: 1996-2001.

In a further example, single molecule, real-time (SMRT) DNA sequencing technology provided by Pacific Biosciences Inc. can be utilized with the methods described herein. In some embodiments, a SMRT chip or the like may be utilized. A SMRT chip comprises a plurality of zero-mode waveguides (ZMW). Each ZMW comprises a cylindrical hole several nanometers in diameter perforating a thin metal film supported by a transparent substrate. When the ZMW is illuminated through the transparent substrate, attenuated light may penetrate the lower than 20-30 nm of each ZMW. Smaller detection volumes may increase the sensitivity of detecting fluorescent signals by reducing the amount of background that can be observed. SMRT chips and similar technology may be used in association with nucleotide monomers fluorescently labeled on the terminal phosphate of the nucleotide. The label is cleaved from the nucleotide monomer on incorporation of the nucleotide into the polynucleotide. Accordingly, the label is not incorporated into the polynucleotide, increasing the signal: background ratio. Moreover, the need for conditions to cleave a label from a labeled nucleotide monomers is reduced.

An additional example of a sequencing platform that is envisaged by the present invention is tSMSYM, i.e. Helicos' Heliscope technology, wherein fragments are captured by polyT oligomers tethered to an array, i.e. a microarray based sequencing method. In each sequencing cycle, polymerase and single fluorescently labeled nucleotides are added and the array is imaged. The fluorescent tag is subsequently removed and the cycle is repeated. The approach accordingly starts with the preparation of a library of target nucleic acids which can be obtained by the addition of a 3' poly(A) tail to each target nucleic acid. The poly(A) tail hybridizes to poly(T) oligonucleotides anchored on a glass cover slip. The poly(T) oligonucleotide can be used as a primer for the extension of a polynucleotide complementary to the target nucleic acid. In one embodiment, fluorescently-labeled nucleotide monomer, i.e. A, C, G, or T, are delivered one at a time to the target nucleic acid in the presence DNA polymerase. Incorporation of a labeled nucleotide into the polynucleotide complementary to the target nucleic acid is detected, and the position of the fluorescent signal on the glass cover slip indicates the molecule that has been extended. The fluorescent label may be removed before the next nucleotide is added to continue the sequencing cycle. Tracking nucleotide incorporation in each polynucleotide strand can provide sequence information for each individual target nucleic acid.

A further envisaged sequence approach is sequencing by combinatorial probe-anchor ligation (cPAL). In embodiments using cPAL, about 10 contiguous bases adjacent to an adaptor may be determined. A pool of probes that includes four distinct labels for each base (A, C, T, G) is used to read the positions adjacent to each adaptor. A separate pool is used to read each position. A pool of probes and an anchor specific to a particular adaptor may be delivered to the target nucleic acid in the presence of ligase. The anchor may hybridize to the adaptor, and a probe hybridizes to the target nucleic acid adjacent to the adaptor. The anchor and probe are ligated to one another. The hybridization is detected and the anchor-probe complex is removed. A different anchor and pool of probes is delivered to the target nucleic acid in the presence of ligase.

Also envisaged are sequencing by hybridization techniques, microscopy-based sequencing techniques, microfluidic Sanger sequencing, or microchip-based sequencing methods.

Particularly preferred are next generation sequencing methods or high throughput sequencing methods as defined herein.

Further details regarding sequencing techniques and approaches may also be derived, for example, from Shendure and Hanlee, 2008, Nat. Biotech, 26:1135-1145.

The present invention also envisages further developments of these techniques, e.g. further improvements of the accuracy of the sequence determination, or the time needed for the determination of the genomic sequence of an organism etc.

In a particularly preferred embodiment of the present invention, the identification of the RNA molecules directly or indirectly associated with at least one ribosome as described herein comprises the determination of the sequence of the RNA. The term "determination of the sequence of the RNA" as used herein refers to sequencing approaches, which start with one or more RNA molecules. In order to obtain such sequences all suitable sequencing techniques may be used. For instance, techniques as described in Ozsolak and Milos, 2011, Nature Reviews Genetics, 12, 87-98 may be utilized.

In specific embodiments the RNA molecule as defined herein is first converted to a cDNA molecule. This cDNA molecule may subsequently be sequenced with any suitable DNA sequencing methodology available, e.g. with next-generation sequencing methods as described herein. This RNA-Seq approach typically links cDNA production based on the use of reverse transcriptase with pyrosequencing as provided on the 454 platform or derived models, polymerase-based sequencing as developed by Illumina or ligation based sequencing as possible on the SOLiD sequencing platform.

In further preferred embodiments, the RNA molecule as defined herein is directly sequenced. An envisaged example of such an approach is the DRS technique based on Helicos single-molecule sequencing platform. This technique essentially relies on hybridization of several femtomoles of 3'-polyadenylated RNA templates to single channels of poly(dT)- coated sequencing surfaces, followed by sequencing by synthesis. This approach is in particular suitable in order to select and sequence mRNA or poly(A)+ RNA, with sequence data being derived from regions immediately upstream of the polyadenylation sites. RNA species that lack natural poly(A) tails, e.g. microRNAs etc. can also be sequenced by polyadenylating them in vitro with subsequent analysis of DRS. DRS sample preparation involving polyadenylation can, in principle, be applied to any RNA species, thus allowing both short and long RNAs to be observed in a single experiment. DRS may further be used in targeted RNA-seq by enabling the integration of target selection and sequencing steps. Such integration may advantageously reduce the sample preparation steps to only nucleic acid fragmentation, and may minimize the quantity of input nucleic acid required. Further details can be derived from suitable literature sources such as Ozsolak and Milos, 2011, Nature Reviews Genetics, 12, 87-98.

Yet another envisaged approach for direct RNA sequencing is based on the NanoString nCounter System, which provides a method for RNA quantification without the requirement for cDNA synthesis. The method relies on the generation of target-specific probes. The probe mixture is typically hybridized to RNA samples in solution, followed by the immobilization of probe-RNA duplexes on surfaces and single-molecule imaging to identify and count individual transcripts. The system may detect several thousand transcripts simultaneously. This approach typically requires amounts of about 100 nanograms of RNA. Further details can be derived from suitable literature sources such as Ozsolak and Milos, 2011, Nature Reviews Genetics, 12, 87-98.

In further specific embodiments, the present invention envisages the sequencing of RNA molecules obtained according to the methods of the present invention without previous amplification of the molecules. Such approaches may, for example, be based on the sequencing of first-strand cDNA products from as little as about 500 picograms of RNA, with priming carried out in solution with oligo-dT or random hexamers. Another approach involves the use of poly(dT) primers on sequencing surfaces to select for poly(A)+ mRNA, followed by onsurface first-strand cDNA synthesis and sequencing. This approach essentially eliminates RNA loss during the RNA isolation steps. Further details can be derived from suitable literature sources such as Ozsolak and Milos, 2011, Nature Reviews Genetics, 12, 87-98.

The term "ribosome" as used herein refers a complex of ribosomal RNA and ribosomal proteins. Each ribosome is typically divided into two subunits. The smaller subunit binds to mRNA molecules, while the larger subunit binds to tRNA molecules and amino acids. Ribosomes from bacteria, archaea and eukaryotes typically differ in their size, sequence, structure, and the ratio of protein to RNA. Prokaryotes typically have 70S ribosomes, each consisting of a small (30S) and a large (50S) subunit. Their small subunit typically has a 16S RNA subunit bound to 21 proteins. The large subunit is typically composed of a 5S RNA subunit, a 23S RNA subunit and 31 proteins. Eukaryotes have 80S ribosomes, each consisting of a small (40S) and large (60S) subunit. Their 40S subunit has a 18S RNA and 33 proteins. The large subunit is typically composed of 5S RNA, 28S RNA, and 5.8S RNA subunits and about 49 proteins. The ribosomes found in mitochondria of eukaryotes typically consist of large and small subunits bound together with proteins into one 70S particle. A ribosome according to the present invention may accordingly be a eukaryotic, prokaryotic, archaea, or mitochondrial ribosome. The ribosome may further be the entire complex as mentioned above, or only a sub-complex thereof, e.g. only a large or small subunit of the ribosomal complex, or a sub-complex of the large or small subunit of the ribosomal complex. The association of an RNA molecule with a ribosome may take place at any position of the ribosome or its sub-complexes, which are accessible during typical operation steps of a ribosome. A ribosome may thus also be one or more ribosomal proteins and/or one or more ribosomal RNA molecules typically forming at least part of a ribosomal complex involved in translational protein or peptide synthesis. If reference is made to an association with at least one ribosome, the ribosome may also be at least one ribosomal protein or at least one ribosomal RNA molecule typically found in a prokaryotic, eukaryotic, archaea or mitochondrial ribosome.

The term "at least one ribosome" refers to the fact that ribosomes may be present on a transcript RNA as single ribosomes or in the form of multiple ribosomes, e.g. as polysomes. The present invention accordingly envisages the association of RNA molecules with a single ribosome, as well as the association with more than one ribosome, e.g. with 2, 3, 4, 5, 6, 7, 8, 9, 10, 15, 20 or more than 20 ribosomes.

The term "cellular extract" as used herein refers to the product or end result of a disruption procedure carried out on a prokaryotic, eukaryotic or archaea cell, or a group of such cells, or on a tissue, an organ, or an entire organism. The disruption procedure may be carried out according to conventional methods known in the art. These methods may be adapted to the cell type, cell size, amount of cells, tissue form, tissue type or further parameters known to the skilled person. For example, easily disrupted cells such as blood cells, insect or animal cells which are grown in culture media, a mild osmosis-based method for cell disruption, i.e. a lysis may be used. Accordingly, the lowering of the ionic strength of a medium media, may be employed to cause the cells to swell and subsequently burst. The approach may further include the addition of a mild surfactant and/or some mechanical agitation to enforce the dissociation of cellular components. Further extraction methods include the use of enzymatic methods to remove cell walls. The enzymes to be used can typically be derived from biological sources, e.g. snail gut or hen egg white. Examples of enzymes which may be used include lysozyme, lysostaphin, zymolase, cellulase, mutanolysin, glycanases, proteases, and mannase. A further extraction method includes cell disruption by employing small glass, ceramic or steel beads mixed with a sample suspended in aqueous media. This beadbeating method may be employed several cellular materials or tissues including spores, animal and plant tissues. A further method which may be used in order to provide cellular extracts is sonication, which is based on the use of ultrasound (e.g. of 20-50 kHz) to the sample. Typically, the high-frequency is generated electronically and the mechanical energy is transmitted to the sample via a metal probe that oscillates with high frequency. The metal probe may be placed into a cell-material containing sample and high-frequency oscillation may cause a localized low pressure region resulting in cavitation and impaction, ultimately breaking open the cells. Another possibility is the use of detergents which lead to cell lysis. Detergents typically disrupt the lipid barrier surrounding cells by disrupting lipid:lipid, lipid:protein and protein:protein interactions. Animal cells, bacteria and yeasts may have differing requirements for optimal lysis due to the presence or absence of a cell wall. Typically, due to the dense and complex nature of animal tissues, they require both detergent and mechanical lysis to effectively lyse cells. For this approach nonionic and zwitterionic detergents such as CHAPS or Triton, Tween or NP-40, and ionic detergents such as SDS may be used.

Another method for providing cellular extracts envisaged by the present invention is the rapid decompression of cells. In this process, cells in question are placed under high pressure (usually nitrogen or other inert gas up to about 25,000 psi) and the pressure is rapidly released. The rapid pressure drop may cause the dissolved gas to be released as bubbles that ultimately lyse the cell.

Further envisaged disruption procedures include high-shear mechanical cell disruptions by using rotor-stator disruptors, valve-type processors, fixed-geometry processors and fixed orifice and constant pressure processors. Furthermore, the cellular extraction may comprise traditional grinding procedures. For example, a cell, tissue, organ etc. may be frozen in liquid nitrogen and subsequently ground by using a mortar or the like. Subsequently, an extraction solvent may be added and the mixture may be stirred. Alternatively, cells or tissues may be ground by means of an impact or cutting, and then added with an extraction solvent and stirred. Further steps such as liquid collection by centrifugation or the like, and purification, e.g. through a gel filtration etc may follow.

Accordingly obtained cellular extracts may be further processed, e.g. by the addition of suitable buffers or stabilizing compounds etc. Typically, the cellular extraction process is accompanied by activities aiming at preserving the integrity of RNA molecules. Such activities may include the use of low temperatures and/or the employment of RNase inhibitors during the extraction process. Furthermore, the cellular extraction process may be accompanied by activities aiming at the termination of protein production in a cell. Accordingly, the addition of cycloheximide, which is an inhibitor of protein biosynthesis in eukaryotic organisms and exerts its effect by interfering with the translocation step in protein synthesis and thus blocking translational elongation, or of functionally equivalent compounds is envisaged. In further embodiments eukaryotic organisms, cells or tissues may be collected and/or frozen in suitable buffers comprising cycloheximide. It is particularly preferred that cycloheximide be used in the context of eukaryotic organisms, cells or tissues. It is further preferred that cycloheximide is not used in the context of prokaryotic organisms or cells, or archaea.

According to specific embodiments of the present invention a cellular extract may be a whole cell or whole tissue or whole organ extract. Alternatively, the cellular extract may comprise a sub-fraction of the originally obtained disruption products. For example, certain cellular compounds or structures may be removed from the cellular extract, e.g. cell walls, membranes, microtubuli, DNA molecules, certain proteins or peptides etc. Such a separation may be carried out according to conventional methods known to the skilled person, e.g. centrifugation, column separation, magnetic particle separation, filtration etc.

In specific embodiments of the present invention, the cellular extract is an extract derived from a eukaryotic cell. The extract may also be derived from an archaebacterial cell. Further envisaged is that the extract be derived from a prokaryotic cell. The extract derived from a eukaryotic cell may preferably be a mammalian cell extract, more preferably a human cell extract. It is also envisaged that the extract be derived from a mitochondrium within a eukaryotic cell, in particular from a mitochrondrium of a mammalian cell, or more preferably from a mitochondrium of a human cell. Moreover, it is envisaged that the cellular extract is an extract derived from the cytoplasm of a eukaryotic cell. Suitable methods for cultivating these cells and for adapting protocols as described herein above to the specific cell type or cell form would be known to the skilled person. In further embodiments, the cellular extract may be a tissue extract, preferably from a eukaryotic organism, more preferably from a mammalian organism, even more preferably from a human being. In case of an animal, in particular mammalian or human organism, cells to be disrupted or cell extracts may also be derived from bodily fluids or tissue biopsies. Such bodily fluids include, for example, whole blood, serum, plasma, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, liquid stool, fluid harvested from a site of an immune response, fluid harvested from a pooled collection site, bronchial lavage, or urine. In further embodiments also material such as biopsy material, e.g. from all suitable organs, e.g. the lung, the muscle, brain, liver, skin, pancreas, stomach, heart etc., a nucleated cell sample, a fluid associated with a mucosal surface, hair, or skin may be used. In order to be extracted, the biopsy material is typically homogenized and/or resuspended in a suitable buffer solution as described herein above.

In a specific embodiment of the present invention the cellular or tissue extract is derived from cells or tissues grown under several physiological, environmental, chemical, physical, or medical conditions or obtained from cells or tissues grown under several physiological, environmental, chemical, physical, or medical conditions. These conditions may be any suitable conditions which are medically, biochemically, biologically, physiologically, toxicologically, diagnostically, or therapeutically relevant.

Examples of envisaged conditions under which cells or tissues are grown include metabolic stress conditions. Such stress conditions may be caused by glucoses level increases or decreases, hormone and other signaling factor level increases or decreases, light increases or decreases, mechanic strength increases or decreases. Further conditions are specific nutrient and/or supply conditions such as the growth of cells or organisms under defined nutrient conditions, e.g. reduced or increased nitrogen supply, reduced or increased carbohydrate supply, reduced or increased phosphate supply, the presence or absence of specific sugar molecules or types in nutrition, the presence or absence of fatty acids in nutrition, the presence or absence of vitamins or enzymatic cofactors in nutrition, and/or the presence or absence of one or more amino acids in the nutrients of an organism or cell. Further envisaged conditions are conditions involving the presence or absence of one or more growth factors such as Adrenomedullin (AM), Angiopoietin (Ang), Autocrine motility factor, Bone morphogenetic proteins (BMPs), Brain-derived neurotrophic factor (BDNF), Epidermal growth factor (EGF), Erythropoietin (EPO), Fibroblast growth factor (FGF), Glial cell line-derived neurotrophic factor (GDNF), Granulocyte colony-stimulating factor (G-CSF), Granulocyte macrophage colony-stimulating factor (GM-CSF), Growth differentiation factor-9 (GDF9), Hepatocyte growth factor (HGF), Hepatoma-derived growth factor (HDGF), Insulin-like growth factor (IGF), Migration-stimulating factor, Myostatin (GDF-8), Nerve growth factor (NGF) and other neurotrophins, Platelet-derived growth factor (PDGF), Thrombopoietin (TPO), Transforming growth factor alpha (TGF-a), Transforming growth factor beta (TGF-β), Tumor-necrosis-factor-alpha (TNF-a), Vascular endothelial growth factor (VEGF), Wnt Signaling molecules, placental growth factor (PIGF), or FBS.

Further envisaged conditions are conditions involving the presence or absence of one or more differentiation factors such as Growth differentiation factors (GDFs) 1 to 15, DIF, LIF, RANKL, Hemopoietic growth factors, ODG or Neurod1.

Further envisaged conditions are conditions involving the presence or absence of one or more receptor ligands such as the ligands of the receptors of the groups of G protein-linked receptors, ion channel-linked receptors, enzyme-linked receptors.

Further envisaged conditions are conditions involving the presence or absence of pharmaceutically active compounds. The term "pharmaceutically active compounds" as used herein refers to any small molecule, protein or peptide, antibody or other chemical or biological entity in the form of an active ingredient, which are known to exert a biological function on an organism, a part of an organism, a cell or tissue. The biological function may include more than one physiological, biochemical or medical effect. The pharmaceutically active compound may be provided as such, i.e. as active ingredient, or it may be provided in the form of a formulation, e.g. with an excipient etc. Pharmaceutically active compounds may be derived, for example, from libraries of known modulators or FDA approved drugs. Examples of such libraries are the Library of Pharmacologically Active Compounds (LOPAC1280), the NIH Clinical Collection (NIHCC), the NCI DTP, the Microsource Spectrum, and the Biomol/Enzo Life Sciences ICCB Known Bioactives and FDA Approved Drug Library.

Further envisaged conditions are conditions involving the presence or absence of potentially pharmaceutically active compounds. The term "potentially pharmaceutically active compounds" as used herein refers to any small molecule, protein or peptide, antibody or other chemical or biological entity in the form of an active ingredient, which are assumed to provide a biological function on an organism, a part of an organism, a cell or tissue. The biological function may include more than one physiological, biochemical or medical effect. The assumption may be a general assumption, or may be based on previous knowledge of the compound class, substituents, form and size, chemical or physical parameters etc. of the compound. The potentially pharmaceutically active compound may be provided as such, or it may be provided in the form of a formulation, e.g. with an excipient etc. The potentially pharmaceutically active compound may be derivable from an experimental compound library or chemical library such as, for example, Enzo Life Science's library, ChemDiv library, the SPECS library or the Chembridge library.

Further envisaged conditions are toxic conditions. Such toxic conditions may be due to the presence of known toxic compounds. Known toxic compounds may be derived from databases such as the database of the US Department of Labor, or the database of the US Environmental Protection Agency. Examples of such toxic compounds include, in particular, carcinogenic compounds or metabolic toxins. Toxic compounds may be derivable from a compound library or chemical library such as, for example, Enzo Life Science's library, ChemDiv library, the SPECS library or the Chembridge library.

Further envisaged conditions are potentially toxic conditions. Such potentially toxic conditions may be due to the presence of chemical or biological compounds, which are assumed to have toxic effects on the organism, tissue, organ or cell. Potentially toxic compounds may be derivable from a compound library or chemical library such as, for example, Enzo Life Science's library, ChemDiv library, the SPECS library or the Chembridge library.

Further envisaged conditions are pathological conditions. Such pathological conditions may be due to the presence of chemical or biological compounds, which are known to be involved in medical manifestations such as injury, necrosis, inflammation, absence of wound healing, neoplasia or any other disease animals, plants, in particular mammals, preferably in humans.

Further envisaged conditions are potentially pathological conditions. Such pathological conditions may be due to the presence of chemical or biological compounds, which are assumed or speculated to be involved in medical manifestations such as injury, necrosis, inflammation, absence of wound healing, neoplasia or any other disease animals, plants, in particular mammals, preferably in humans.

Further envisaged conditions are one or more stages or phases of development and/or differentiation of an organism, organ, tissue or cell. For instance the condition may be related to a specific phase or stage of a development or differentiation of an organism, organ, tissue or cell. This condition may further be compared to a different, second specific phase or stage of a development or differentiation of an organism, organ, tissue or cell. Comparisons may also be carried out with normal or typical or known developmental or differentiation stages, phases or pattern. For example, the state of a fully developed or differentiated organ, cell, organism etc. may be used for comparison purposes.

Also envisaged is a condition relating to one or more phases or stages of stem-cell differentiation. For example, stem-cells may be used as bases for acquiring ribosomal bound RNAs in extracts as defined herein during stem-cell development or differentiation. The accordingly identified RNA molecules or species may be compared with molecules obtained in differentiated cells, in one or more different phases or stages of stem-cell development, or in certain or specific cells types or tissue types. The developmental stages may, in additional embodiments, also be tightly monitored, e.g. during specific time frames, or during certain phases of development etc.

Further examples of envisaged conditions under which cells or tissues are grown include physical or chemical conditions such as temperature, e.g. an increased or reduced temperature in comparison to a normal physiological situation, pH, e.g. an increased or reduced pH in comparison to a normal physiological situation, light, e.g. an increased or reduced exposition to light in comparison to a normal physiological situation, movement, e.g. an increased or reduced movement of cells, organism etc. in comparison to a normal physiological situation, or for example an increased or reduced mechanic strength or stress in comparison to a normal physiological situation The term "normal physiological situation" as used herein refers to physical or chemical conditions which are typically encountered in the natural habitat of an organism or cell etc.

Further examples of envisaged conditions under which cells or tissues are grown include normal, healthy or optimal conditions. The term "normal condition" as used herein describes a situation in which the organism, tissue, organ, or cell is not affected by extreme conditions, or is provided within the range of typical physiological, environmental, chemical, or physical parameters, e.g. mechanical impact on cells or tissues. The term "healthy condition" as used herein describes a situation in which the organism, tissue, organ, or cell is not affected by toxic, pathological or disease-causing factors, e.g. as defined herein above. The term "optimal condition" as used herein describes a situation in which the organism, tissue, organ, or cell is provided with physiological, environmental, chemical, or physical parameters which are in their optimal range, e.g. leading to optimal functioning of the organism, tissue, organ or cell. The optimal range of parameters is understood as being narrower than the typical/normal range of parameters. Corresponding information would be known to the skilled person or can be derived from suitable literature sources.

In specific embodiments it is envisaged that any two or more of the above mentioned conditions are compared in the context of an organism, tissue, organ or cell. For example, an organism, tissue, organ or cell may be grown under conditions involving the presence of a pharmaceutically active compound (condition 1), while a further organism of the same species, an identical tissue or organ or a cell derived from the same organism or derived from the same tissue or from a cell culture, or being a clone of the above mentioned cell is grown under a different condition, e.g. a healthy, normal or optimal condition (condition 2). Any other combination of conditions as outlined above is also possible, e.g. toxic condition (condition 1) vs. healthy condition (condition 2), presence of growth factors (condition 1) vs. differences in nutrient supply (condition 2) etc. It is in particular envisaged that cellular or tissue extracts may be provided from such organism, tissues, organs or cells and subsequently RNA be identified according to the method of the present invention. RNA molecules identified under such differing conditions may subsequently be compared. Such a comparison may be carried out in silico, e.g. with the help of suitable software tools as known to the skilled person, or by specific hybridization protocols. It is envisaged by the preset invention that such an approach leads to the identification of differentially present RNA molecules, e.g. RNA species which are present under condition 1, but not under condition 2, or vice versa, or of RNA species which are present under both conditions. It is envisaged by the preset invention that such an approach leads to the identification of differentially present RNA molecules, e.g. RNA species which are present under both conditions, but at different expression levels. A further comparison of more than two conditions is also envisaged, e.g. a comparison of 3, 4, 5, 6, 7, 8, 9, 10 or more conditions in terms of presence of RNA molecules.

The term "contacting" as used refers to the provision of physical interaction between two entities, in particular a cellular extract and a molecule binding to a ribosomal protein. The contacting may be achieved, for example, by mixing a liquid extract and a liquid comprising said binding molecule or a matrix, bead or substrate comprising said binding molecule in immobilized form, or coated with such a binding molecule, such that the binding molecule is provided in sufficient amounts and mixed so thoroughly that an effective binding, i.e. interaction, between ribosomes or ribosomal proteins comprised in said extract and said binding molecule becomes possible. In certain embodiments, the binding molecule may or may not be coupled to a matrix. The concentration of binding molecules, its confirmation and the details of the mixing step are known to the skilled person and/or can be adapted to the specific situation or protocol in use. The contacting may further comprise the use of additional assisting elements, e.g. the employment of magnetic forces, the provision of liquid or air flows, the increase or decrease of temperature, the change or set of pH, ionic concentrations or detergents etc.

The method as described above further preferably refers to a specifically binding molecule, which is immobilized on a substrate, matrix or bead.

The term "immobilization" as used herein refers to the association of a binding molecule to a support material via molecular interactions which position the binding molecule at a specific area of the support material and concomitantly impede a detaching of the binding molecule, e.g. during further treatment steps, such as washing or rinsing steps etc. Typically, such molecular interactions are based on covalent chemical bonds or covalent chemical interactions between structural elements or functional groups of the support material and the molecule to be immobilized, e.g. corresponding functional groups of the molecule, as known to the person skilled in the art. Also envisaged is an immobilization or attachment of the binding molecule to a support material by non-covalent interactions. The immobilization may, for example, be carried out by crosslinking the binding molecule by heat or light, i.e. by forming molecular interactions or bonds that link both structural elements together under the influence or driven by the energy provided by an energy source like heat or light, or via a chemical immobilization. Typically, an immobilization via crosslinking by heat is carried out via drying and subsequent baking or heating of binding molecules on a support material at certain temperatures. Drying and baking are believed to result in molecules becoming attached to the support material by hydrophobic interaction. This procedure can be classified as a sub-form of physical adsorption. The term "physical adsorption" relates to a process involving initial separation and attraction steps, whereby the binding molecule comes into proximity with the reactive groups, which are based on physical adsorptive processes. The adsorption of a biomolecule, e.g. a protein or nucleic acid, onto a support material may take place with practically any support material, since it has been observed that any such support material will interact with almost any surface. Typically, the level of interaction between support material and binding molecules to be immobilized varies depending on the nature and form of the support material and the size and chemical properties of the binding molecules. The interaction is typically a five-stage procedure, comprising the steps of (i) transport of the binding molecule to the surface, (ii) adsorption to the surface, (iii) rearrangement of the adsorbed binding molecule, (iv) potential desorption of the adsorbed binding molecule and (v) transport of the desorbed binding molecule away from the surface. Although the procedure implies, to a certain extent, that the potential for desorption is inherent, the binding is typically irreversible, depending on size of the binding molecule. The term "size of the binding molecule" within the context of adsorption interactions relates to the number of binding sites that are present. Although any one binding site may, in principle, dissociate from support material at any time, the effect of a large number of binding sites is that the binding molecule as a whole will remain bound. By applying energy in the form of heat, e.g. at a temperature of about 40 to 150°C, the physical adsorption of the binding molecule to the support material may be enhanced and the time necessary for an efficient immobilization may be shortened. The crosslinking by heat may be carried out for any suitable period of time known to the person skilled in the art, e.g. 2 min to 12 hours, preferably 10 min to 8 hours. The crosslinking by heat or baking may be carried out by any suitable means known to the person skilled in the art, for example a drying chamber or an oven. In addition to the temperature, also other parameters like humidity, aeration or ventilation may be adjusted to suitable values known to the person skilled in the art. The crosslinking by heat or baking may also be combined with other forms of immobilization like crosslinking by light or chemical immobilization.

Crosslinking by light is typically performed by applying light of a typical wavelength, e.g. in a range of 150 to 550 nm, preferably in a range of 200 to 500 nm to binding molecules in order to induce an interaction between the binding molecules and support material. Typically, the induced interaction between the binding molecules and the support material is a covalent binding of the binding molecule to the material. Crosslinking by light may, for example, be carried out by using UV light. UV crosslinking is one of the simplest ways to ensure covalent binding of a support material to a binding molecule. In the case of nucleic acids, the linkage proceeds through the bases of a nucleic acid molecule, e.g. thymine, guanine, adenine, cytosine or uracil residues, which react with corresponding and suitable functional chemical groups on the support material, as known to the person skilled in the art.

A "chemical immobilization" as mentioned herein may be an interaction between the support material and the binding molecule based on chemical reactions. Such a chemical reaction can be enhanced by either applying heat, e.g. a certain optimal temperature for a chemical reaction. For example, a chemical immobilization may take place between functional groups on a support material and corresponding functional elements on the binding molecules. Such corresponding functional elements in the binding molecules may either be as part of the chemical inventory of a molecule, or be additionally introduced.

An example of such a functional group is an amine group. Typically, the binding molecule to be immobilized, e.g. a nucleic acid or protein, comprises a functional amine group or is chemically modified in order to comprise a functional amine group. For example, the amino group may react with activated groups on the support material, such as a carboxy activated group. Examples include carbonyl, epoxy or similar groups. Means and methods for such a chemical modification are known to the person skilled in the art. The localization of said functional groups within the binding molecule to be immobilized may be used in order to control and shape the binding behavior and/or orientation of the binding molecule. A typical reaction partner for a binding molecule to be immobilized comprises moieties which are capable of binding to such binding molecules such as amine-functionalized nucleic acids.

An "immobilization or attachment of the binding molecule to a support material by non-covalent interactions" may comprise the interaction between the support material and the binding molecule by hydrogen bonding, electrostatic interactions, Van der Waals interactions, and/or hydrophobic packing. Envisaged examples of non-covalent immobilization or attachment of binding molecules to the support material include the use of biotin-streptavidin interactions. For example, a binding molecule as defined herein, e.g. a DARPin, may be coupled to a biotin molecule or be biotinylated. Support material as defined herein may accordingly be provided with streptavidin molecules. Alternatively, similar molecules such as avidin, streptavidin derivatives, (strept)avidin, avidin-related proteins, avidin-like entities such as tamavidin 1 and 2, bradavidin, or NeutrAvidin etc. may be used. These molecules may be coupled in a covalent manner to said support material. Subsequently a non-covalent interaction and attachment, e.g. between the biotinylated molecule and the streptavidin comprising material may be performed, resulting in an immobilization of the binding molecule to the support material.

Further examples of non-covalent interactions include the use of protein G or protein A on a support material, e.g. a bead such as a dynabead or magnetic bead, or a matrix or a substrate. Protein G or protein A may accordingly be coupled in a covalent manner to said bead, matrix or substrate. A non-covalent interaction may subsequently be carried out upon the provision of an antibody, which can interact and bind to said protein G or protein A covered support material.

In further embodiments, the presence and number of functional chemical groups on or inside the support material, e.g. a bead, substrate or matrix as defined herein may be controlled and adjusted via suitable chemical activation processes. Such activation processes may, for instance, provide specifically localized functional groups on or within a support material and facilitate a specific interaction between the binding molecules and the material within the context of these localized functional groups. The presence and number of functional group on or inside the support material may also have an influence on the orientation and freedom of the immobilized binding molecules. For example, the presence of a higher number of functional groups may lead to an immobilization at different points within the binding molecule. Furthermore, the presence of corresponding reactive elements within the binding molecule may be used for a control of the orientation of the binding molecule on the support material.

The term "substrate" as used herein refers to any suitable substrate known to the person skilled in the art. The substrate may have any suitable form or format, e.g. it may be flat, curved, e.g. convexly or concavely curved towards a region where interactions takes place, it may be curled or comprise a wavelike format. It may also be organized in round shape structures. Typically, the substrate is a solid support, i.e. comprising support material, which is mainly of non-liquid consistence and thereby allows for an accurate positioning of binding molecule on the support material. An example of a substrate is a solid material or a substrate comprising functional chemical groups, e.g. amine groups or amine-functionalized groups. Further examples of a substrate envisaged by the present invention are carboxylated polystyrene or silica microspheres. A further typical support material or substrate type is a non-porous substrate. Preferred among non-porous substrates are glass, poly-L-lysine coated material, nitrocellulose, cyclic olefin copolymers (COCs), cyclic olefin polymers (COPs), polypropylene, polyethylene and polycarbonate.

A "matrix" as used herein refers to an irregularly shaped support material. The matrix shall preferably comprise no small cavity or hole elements. In certain embodiments, the support may be provided on a solid body of substance or underlying a solid layer. In other embodiments, the entire matrix material may be comprised of cavities or holes, e.g. in the form of a mesh or 3 dimensional lattice. The surface of a matrix may accordingly be inclined, or non-planar with frequent changes or inclinations. Examples of matrix structures include polyvinylalcohol structures, polyethylene structures, chemically modified polysaccharide fibers, non-modified polysaccharide fibers, and mixtures thereof.

The term "bead" as used herein refers to round shaped support or substrate structures. The beads may be organized in arrays or geometrical forms, e.g. located at predefined points or spots, or may be provided in an irregular manner. Beads may further overlay a substrate ground or be fixed on substrate ground by connector elements such as rods etc. An example of bead elements envisaged by the present invention are magnetic particles comprising binding molecules. Beads may further be coated with protective coatings, e.g. PEG molecules, and/or with functionalized coatings, e.g. comprising biochemically active compounds. Preferred beads are dynabeads or similar magnetic beads, e.g. with suitable functional groups such as epoxy groups. Further envisaged examples include carboxyl (COOH) microparticles. Such microparticles may be used for covalent coupling of proteins by activating the carboxyl groups with water-soluble carbodiimide. The carbodiimide may further react with the carboxyl group allowing the creation of an active ester that may be reactive toward primary amines on any protein of interest, e.g. a specifically binding molecule as defined herein. Microparticle coupling may, for example, be carried out with a PolyLink coupling kit as provided by Polysciences Inc. (e.g. as described in Halbeisen et al., PLoS Biol. 2009).

A "molecule specifically binding to a ribosomal protein" as used herein is a molecule, which is capable of binding essentially only one specific ribosomal protein and does not show significant cross-reactivity with other proteins or entities present in the cellular extract. The specific binding may take place at epitopes of a ribosomal protein, e.g. linear or conformational epitopes of a ribosomal protein. The recognized binding site in the ribosomal protein is typically provided within the ribosomal complex as defined herein, i.e. in a natural state of a ribosome. In certain embodiments the specific binding takes place at protein stretches or amino acids present at the surface of a ribosomal protein, or at solvent exposed sites of a ribosome or ribosomal protein. The binding between the molecule and the ribosomal protein may be a high affinity binding in the range of 1 x 10⁻³ to 1 x 10⁻⁹ mM. The binding may further be sufficiently tight as to allow for immunopurification or immunoprecipitation of the bound ribosome. By specifically binding a ribosomal protein a ribosome complex comprising said ribosomal protein may thus be attracted to a substrate, matrix or bead as defined herein. This specific binding, which does not require any manipulation or modification of the ribosomal protein or ribosome structure such as the expression and presence of tag on the ribosomal proteins, accordingly allows to retain ribosomes and therewith associated RNA molecules in virtually any cellular extract available. The associated RNA molecules are typically mRNA molecules or other RNA molecules which are transiently bound to the ribosome. Subsequent steps may be used to safeguard the RNA molecules and/or to determine the identity of those RNA molecules, which are retained together with the ribosomal complex due to their association therewith. In certain embodiments of the present invention, a binding molecule as defined herein may bind to more than one ribosomal protein at the same time. The binding molecule may, for example, simultaneously bind to two or three ribosomal proteins. Such a scenario could reflect a conformational or 3D epitope being provided by more than one ribosomal protein, e.g. by two, three or more ribosomal proteins.

A "molecule specifically binding to a group of ribosomal proteins" as used herein is a molecule, which is capable of binding several specific ribosomal proteins at the same time and does not show significant cross-reactivity with other non-ribosomal proteins or entities present in the cellular extract. The specific binding may take place at different epitopes of several ribosomal proteins, e.g. linear or conformational epitopes of several ribosomal proteins. The recognized binding sites in the group of ribosomal proteins is typically provided within the ribosomal complex as defined herein, i.e. in a natural state of a ribosome. In certain embodiments the specific binding takes place at protein stretches or amino acids present at the surface of a group of ribosomal protein, or at solvent exposed sites of a ribosome complex. The binding between the molecule and the group of ribosomal proteins may be a high affinity binding in the range of 1 x 10⁻³ to 1 x 10⁻⁹ mM. The binding may further be sufficiently tight as to allow for immunopurification or immunoprecipitation of the bound ribosome. By specifically binding a group of ribosomal proteins a ribosome complex comprising said group of ribosomal proteins may thus be attracted to a substrate, matrix or bead as defined herein. This specific binding, which does not require any manipulation or modification of the group of ribosomal proteins or ribosome structure such as the expression and presence of a tag on the ribosomal proteins, accordingly allows to retain ribosomes and therewith associated RNA molecules in virtually any cellular extract available. The associated RNA molecules are typically mRNA molecules or other RNA molecules which are transiently bound to the ribosome. Subsequent steps may be used to safeguard the RNA molecules and/or to determine the identity of those RNA molecules, which are retained together with the ribosomal complex due to their association therewith.

Specifically binding molecules as defined herein are provided in a varying amount and concentration on a substrate, matrix or bead. For example, the binding molecules may be present in a range of 10² to 10⁸ molecules per interaction zone. The number of binding molecules may be adapted to the number of cells present. For example, in order to capture the 200.000 to 3 million ribosomes which are typically present in a eukaryotic cell, the number of binding molecules provided in an interaction zone may be about 200.000 to 3 million multiplied by the number of cells used for the analysis. This number may be varied according to further parameters such as the cell type, the origin of the cell, the physiological conditions used, the intention of the experiment etc. Further numbers of binding molecules per interaction zone are also envisaged, e.g. less binding molecules than ribosomes present, or more binding molecules than ribosomes present etc.

In one embodiment of the invention, only one type of binding molecule, i.e. a molecule binding specifically to only one ribosomal protein or part of the ribosome complex is present on the substrate, matrix or bead. By providing such an array of molecules it is considered possible to bind and subsequently immobilize to the substrate all ribosomes, or essentially all ribosomes, e.g. about 99%, 98%, 95%, 90%, or 85% of the ribosomes present in a cellular extract which was brought in contact to the substrate. In further embodiments, only a portion of all ribosomes present in a cellular extract may be bound, e.g. a portion of 10%, 20%, 30%, 40%, 50%, 60%, 70% or 80%. Since ribosomes are assumed to be engaged in the translation of different RNA molecule types, by binding and subsequently immobilizing all or essentially all ribosomes also all or essentially all therewith associated RNA molecules are bound and brought to the substrate.

In embodiments of the invention the binding of all or essentially all ribosomes to the specific binding molecules may lead to a corresponding pulling down or indirect association with the substrate, matrix or bead of all or essentially all mRNA molecules and/or non-coding RNA molecules which are directly associated with said ribosomes. These mRNA and/or non-coding RNA molecules may subsequently be identified according to the methods of the present invention.

In further embodiments of the invention, the binding of all or essentially all ribosomes to the specific binding molecules may lead to a corresponding pulling down or indirect association with the substrate, matrix or bead of all or essentially all non-coding RNA molecules which are indirectly associated with said ribosomes. These non-coding RNA molecules may subsequently be identified according to the methods of the present invention.

By pulling down directly associated mRNA and/or non-coding RNA molecules also indirectly associated RNA molecules (e.g. microRNAs) may be pulled down due to their association with the directly associated RNA molecules. In certain embodiments, the linking between the directly and the indirectly associated RNA molecules may be disrupted, e.g. by specific washing, rising or agitation steps in order to only obtain directly associated RNA molecules. Further envisaged is, in specific alternative embodiments, the use of RNase activity in order to remove all RNA molecule fragments which are not covered by ribosomal complex entities and thus protected against RNase activity.

In further embodiments a specific retention of only indirectly associated RNA molecules is achieved by separating said molecules form the directly bound RNA molecules and by subsequently collecting said RNA molecules, optionally followed by their identification via sequencing etc. as described herein. The separation may, for example, be carried out by enriching mRNA molecules via oligo-dT selection. ncRNAs may be enriched according to suitable short RNA enrichment procedures, e.g. by isolation procedures containing phenol and by a suitable combination of a matrix and binding solutions and eluants leading to a very low RNA-length cutoff.

In specific embodiments of the present invention, the method as defined herein above may include a specific binding of more than one interacting pair of molecules and ribosomal epitopes or proteins, i.e. two or more different specifically binding molecules may be used to bind two or more cognate epitopes on ribosomal proteins or ribosomal antigens, e.g. one or more antibodies and one or more DARPins. Such differently bound, independent epitopes may be present on one ribosomal protein, or they may be present on different ribosomal proteins. It is particularly preferred that such differently bound, independent epitopes are at least present on one ribosomal complex, e.g. typical for a specific organism or organism group. In specific embodiments, binding molecules of different origin, which are able to specifically bind to ribosomal proteins or parts of the ribosomal complex may be provided.

Further envisaged is also a binding of a specifically binding molecule as defined herein to one or more non-protein components of the ribosome, e.g. a specific binding to ribosomal RNA. For example, a specifically binding molecule may be provided which is able to bind a protein component of the ribosome and at the same time a ribosomal RNA molecule, e.g. present in a conformational epitope.

According to the invention, the binding molecule may bind to a eukaryotic ribosomal protein, i.e. to a ribosomal protein encountered in eukaryotic ribosomes. The binding to a eukaryotic ribosomal protein may be exclusive, i.e. the binding molecule may only bind to a eukaryotic ribosomal protein and not bind to a ribosomal protein present in prokaryotic, archaea or mitochondrial ribosomal proteins. In further embodiments, the binding to a eukaryotic ribosomal protein may be non-exclusive such that not only a eukaryotic ribosomal protein, but also a prokaryotic and/or an archaea and/or a mitochondrial ribosomal protein can be bound.

The group of eukaryotic ribosomal proteins comprises the ribosomal proteins of any eukaryotic organism known to the person skilled in the art. Within the group of eukaryotic ribosomal proteins, i.e. of ribosomal proteins of eukaryotic organisms, the binding of the specifically binding molecule may be exclusive for species, genera, families, orders, or classes of organisms. Thus, ribosomal proteins present in certain eukaryotic species, genera, families, orders, or classes of organisms may be bound, whereas ribosomal proteins present in differing eukaryotic species, genera, families, orders, or classes of organisms may not be bound. In further embodiments, the binding to a ribosomal protein within the group of eukaryotic ribosomal proteins may also be non-exclusive such that ribosomal proteins of various or all species, genera, families, orders, or classes of eukaryotic organisms can be bound.

According to a further embodiment, the binding molecule may bind to a prokaryotic ribosomal protein, i.e. to a ribosomal protein encountered in prokaryotic ribosomes. The binding to a prokaryotic ribosomal protein may be exclusive, i.e. the binding molecule may only bind to a prokaryotic ribosomal protein and not bind to a ribosomal protein present in eukaryotic, archaea or mitochondrial ribosomes. In further embodiments, the binding to a prokaryotic ribosomal protein may be non-exclusive such that not only a prokaryotic ribosomal protein, but also a eukaryotic and/or an archaea and/or a mitochondrial ribosomal protein can be bound.

The group of prokaryotic ribosomal proteins comprises the ribosomal proteins of any prokaryotic organism known to the person skilled in the art. Within the group of prokaryotic ribosomal proteins, i.e. of ribosomal proteins of prokaryotic organisms, the binding of the specifically binding molecule may be exclusive for species, genera, families, orders, or classes of organisms. Thus, ribosomal proteins present in certain prokaryotic species, genera, families, orders, or classes of organisms may be bound, whereas ribosomal proteins present in differing prokaryotic species, genera, families, orders, or classes of organisms may not be bound. In further embodiments, the binding to a ribosomal protein within the group of prokaryotic ribosomal proteins may also be non-exclusive such that ribosomal proteins of various or all species, genera, families, orders, or classes of prokaryotic organisms can be bound.

According to a further embodiment, the binding molecule may bind to an archaea ribosomal protein, i.e. to a ribosomal protein encountered in archaea ribosomes. The binding to an archaea ribosomal protein may be exclusive, i.e. the binding molecule may only bind to an archaea ribosomal protein and not bind to a ribosomal protein present in prokaryotic, eukaryotic, or mitochondrial ribosomal proteins. In further embodiments, the binding to an archaea ribosomal protein may be non-exclusive such that not only an archaea ribosomal protein, but also a eukaryotic and/or a prokaryotic and/or a mitochondrial ribosomal protein can be bound.

The group of archeae ribosomal proteins comprises the ribosomal proteins of any archaea organism known to the person skilled in the art. Within the group of archaea ribosomal proteins, i.e. of ribosomal proteins of archaea organisms, the binding of the specifically binding molecule may be exclusive for species, genera, families, orders, or classes of organisms. Thus, ribosomal proteins present in certain archaea species, genera, families, orders, or classes of organisms may be bound, whereas ribosomal proteins present in differing archaea species, genera, families, orders, or classes of organisms may not be bound. In further embodiments, the binding to a ribosomal protein within the group of archaea ribosomal proteins may also be non-exclusive such that ribosomal proteins of various or all species, genera, families, orders, or classes of archaea organisms can be bound.

According to yet another embodiment, the binding molecule may bind to a mitochondrial ribosomal protein, i.e. to a ribosomal protein encountered in mitochondrial ribosomes. The binding to a mitochondrial ribosomal protein may be exclusive, i.e. the binding molecule may only bind to a mitochondrial ribosomal protein and not bind to a ribosomal protein present in prokaryotic, eukaryotic, or archaea ribosomal proteins. In further embodiments, the binding to a mitochondrial ribosomal protein may be non-exclusive such that not only a mitochondrial ribosomal protein, but also a eukaryotic and/or a prokaryotic and/or an archaea ribosomal protein can be bound.

Such a non-exclusive binding as described above may be due to the presence of conserved surface structures or epitopes in the group of eukaryotic, prokaryotic, archaea and/or mitochondrial ribosomal proteins, or in any sub-grouping of the group of eukaryotic, prokaryotic, archaea and/or mitochondrial ribosomal proteins. Likewise, a species, genera, families, orders, or classes specific binding may be due to variations in the primary sequence and/or conformation of ribosomal proteins. The skilled person would be aware of such differences or similarities. Further information can be derived from suitable literature sources such as Pubmed, Google Scholar, etc. or databases such as Ribosomal Protein Gene Database (RPGS), Uniprot, Protein Data Bank (PDB), e.g. RCSB PDB or PDBeurope.

According to the present invention the exclusivity of binding to certain ribosomal proteins may advantageously be used in order to distinguish between ribosomes and therewith associated mRNA molecules or non-coding RNA molecules of different provenience, e.g. in case a cellular extract comprises cells or components of different organisms or different origin, such as eukaryotic and prokaryotic cells, or eukaryotic cells and mitochondria etc. Thus, only ribosomes of specific species, genera, families, orders, or classes could be bound and subsequently corresponding RNA molecules identified. As an example, the identification of prokaryotic RNA molecules in a cellular extract comprising eukaryotic and prokaryotic cellular material can be achieved.

The non-exclusivity of binding to certain ribosomal proteins may on the other hand be used to bind essentially all ribosomal proteins present in a cellular extract essentially irrespective of its origin or provenience. Accordingly, RNA molecules associated with said ribosomal proteins can be identified. This provides the advantage that the use of a single specifically binding molecule, or of a small group of such molecules, allows to bind ribosomal proteins of a broad range of origins. The method of the invention based on this principle can accordingly be used with cellular extracts from a broad range of origins, e.g. eukaryotic, prokaryotic, archaea and mitochondrial extracts, without the necessity of adapting the binding protocol, e.g. with only one central binding molecule.

In a preferred embodiment, the ribosomal protein being bound by the binding molecule is a mammalian ribosomal protein. The term "mammalian ribosomal protein" as used herein refers to any ribosomal protein, which can be found in a mammal. The protein may either be exclusively present in a mammal, or it may be conserved and thus be found in further groups of organisms. In a further particularly preferred embodiment, the ribosomal protein being bound by the binding molecule is a human ribosomal protein. The term "human ribosomal protein" as used herein refers to any ribosomal protein, which can be found in a human being. The protein may either be exclusively present in a human being, or it may be conserved and thus be found in further groups of organisms, e.g. in other mammals, in other animals, in other eukaryotes etc.

In order to be able to analyze human mitrochondria, the present invention also envisages that the ribosomal protein bound by the binding molecule is a mitochondrial ribosomal protein, which can be found in human beings, i.e. a human mitochondrial ribosomal protein. The protein may either be exclusively present in a mitochondrium of human beings, or it may be conserved and thus be found in further groups of organisms, e.g. in other mammals, in other animals, in other eukaryotes etc.

In a further embodiment of the present invention, the ribosomal protein, which can be bound by the specifically binding molecule according to the present invention is a ribosomal protein located at the surface of a ribosome. The term "surface of a ribosome" refers to sections and portions of the ribosomal complex or of ribosomal proteins which are oriented towards the outside of the ribosomal complex. Such sections can potentially be "seen" from the outside, e.g. in a crystallographic model, for instance a model as described in Graifer and Karpova, 2012, Acta Biochim Biophys Sin (Shanghai), 44(4): 281-99; Klinge et al., 2012, Trends Biochem Sci. 37(5): 189-98 or Ramakrishnan, 2011, Science 331(6018): 681-682.

In another specific embodiment, the ribosomal protein which can be bound by the specifically binding molecule according to the present invention is located at the 60 S solvent exposed side of a eukaryotic ribosome. The term "solvent exposed side" as used herein refers to all parts or sections of the 60S ribosomal subunit which are in contact with a solvent, e.g. an aqueous buffer, in a natural state, i.e. in a ribosomal complex as can be found in cells in vivo. The solvent exposed side thus essentially comprises proteins, protein sections and amino acids which are at the surface of the ribosome complex. Further details on the 60S solvent exposed side of a eukaryotic ribosome would be known to the skilled person, and can be derived from suitable literature sources such as Moore, 2001, Biochemistry, 40 (11), 3243-3250; Klinge et al., 2012, Trends Biochem Sci. 37(5): 189-98 or Ramakrishnan, 2011, Science 331(6018): 681-682. In a further embodiment of the present invention, the ribosomal protein which can be bound by the specifically binding molecule according to the present invention is a ribosomal protein which becomes temporarily or conditionally accessible to a specifically binding molecule. The term "temporarily or conditionally accessible" as used herein means that the ribosomal protein may change its conformation or surface identity in reaction to internal or external factors. For example, the surface structure and the presence of epitopes or amino acids on the surface of the ribosomal complex may depend on the activity of the ribosome, its stage of process etc., or on the presence of buffers, ions, external molecules, the temperature of environment etc. Furthermore a conditionally accessible ribosomal protein may not be part of the typical ribosomal core structure, but may become part of the ribosome in dependence of certain biochemical or physiological parameters, e.g. the activity of the ribosome, the activity of cellular pathways etc. An example of such a conditional ribosomal protein is L 29. By providing means for modifying these internal or external parameters surface structures of the ribosomal complex or proteins may be presented, which subsequently may disappear, e.g. upon a further change of parameters.

In a particularly preferred embodiment the ribosomal protein which can be bound by the specifically binding molecule according to the present invention is one of the following proteins: Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A,Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, and Homo sapiens ribosomal protein L38. Information on the identity, size, sequence and further properties of these proteins is known to the skilled person and/or can be derived from suitable literature sources or databases such as the EMBL database, NCBI database, UniProt database, SwissProt database etc. Further information may be derived from the human genome database provided by NCBI with version No. 37.3 as accessible on 10 October 2012.

In a further preferred embodiment the ribosomal protein which can be bound by the specifically binding molecule according to the present invention is a homologue of Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A,Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, or Homo sapiens ribosomal protein L38 in a further eukaryotic species, a prokaryotic species or an archaea species.

The term "homologue" as used in the context of the present invention refers to ribosomal proteins which show a high degree of identity to the human ribosomal proteins as described herein above and which are derived from a different species, e.g. from monkeys, mice, rats, vertebrates, invertebrates, bacteria, plants etc., preferably from a different mammalian species.

The term "high degree of identity" as used herein in the context of ribosomal proteins relates to ribosomal proteins which comprise, or alternatively consist of an amino acid sequence which is at least 70%, 75%, 80%, 85%, 86%, 87%, 88%, 89%, 90%, 91%, 92%, 93%, 94%, 95%, 96%, 97%, 98% or 99% identical to, for example, Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A,Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, or Homo sapiens ribosomal protein L38.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

Whether any particular ribosomal protein is at least 70%, 75%, 80%, 85%, 86%, 87%, 88%, 89%, 90%, 91%, 92%, 93%, 94%, 95%, 96%, 97%, 98% or 99% identical to, for instance, an amino acid sequence of a human ribosomal protein as defined herein above, can be determined conventionally by using known computer programs. A preferred method for determining the best overall match between a query sequence (a sequence of a human ribosomal protein as defined herein above) and a subject sequence, also referred to as a global sequence alignment, can be determined using the FASTDB computer program based on the algorithm of Brutlag et al., 1990, Comp. App. Biosci. 6: 237-245. In an amino acid sequences alignment the query and subject sequences are both amino acid sequences. The result of said global sequence alignment is given in percent identity. Preferred parameters used in a FASTDB amino acid alignment are: Matrix=PAM 0, k-tuple=2, Mismatch Penalty=l, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned may be determined by the results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N-and C-terminal residues of the subject sequence. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Only residue positions outside the N- and C-terminal ends of the subject sequence, as displayed in the FASTDB alignment, which are not matched/aligned with the query sequence are manually corrected. No other manual corrections are to be made for the purposes of the present invention.

Homologues of ribosomal proteins as described herein may further be derived or defined in accordance with the information provided in Ribosomal Protein Gene Database (RPGS), which is, for example, accessible at http://ribosome.med.miyazaki-u.ac.jp/.

In a further embodiment of the present invention a homologue of the human ribosomal proteins as defined herein above may be a protein of a non-human eukaryotic species, a prokaryotic species or an archaea species which shows a high degree of identity, e.g. a degree of 90%, 91%, 92%, 93%, 94%, 95%, 96%, 97%, 98% or 99% identity, or be complete identical in only a fragment or domain of the entire protein. Such a fragment of domain is preferably a fragment or domain which is located at the surface of the ribosome or ribosomal complex, or the solvent exposed side of the ribosome. For instance, a surface domain or stretches of amino acids which are located at the surface of a ribosome or ribosomal complex may show a high degree of identity to the human ribosomal protein as defined herein above, whereas other portions of the protein may not have such a high degree of identity, or be entirely unrelated. The present invention accordingly further envisages that a specifically binding molecule only binds to said highly identical portions of the ribosomal protein.

It is particularly preferred that the ribosomal protein which is bound by the specifically binding molecule according to the present invention is Homo sapiens ribosomal protein L13A, Homo sapiens ribosomal protein L18, Homo sapiens ribosomal protein L22 or Homo sapiens ribosomal protein L26. It is further particularly preferred that the ribosomal protein which can be bound by the specifically binding molecule according to the present invention is a homologue of Homo sapiens ribosomal proteins L13A, L18, L22 or L26.
According to a preferred embodiment of the present invention the specifically binding molecule is an antibody, intrabody, antibody substructure, a modified antibody, or a fragment thereof.

The term "antibody" as used herein refers to immunoglobulin molecules and immunologically active portions of immunoglobulin molecules, i.e. molecules that contain an antigen binding site that immunospecifically binds an antigen, in particular specifically binds a ribosomal protein as defined herein above. The immunoglobulin molecules of the invention can be of any type (e.g., IgG, IgE, IgM, IgD, IgA and IgY), class (e. g., IgG1, IgG2, IgG3, IgG4, IgA1 and IgA2) or subclass of immunoglobulin molecule. In a specific embodiment the antibody or fragment thereof as mentioned herein above comprises a human IgM heavy chain constant domain, a human IgG1 heavy chain constant domain, a human IgG2 heavy chain constant domain, a human IgG3 heavy chain constant domain, a human IgG4 heavy chain constant domain, or a human IgA heavy chain constant domain. In a further embodiment of the present invention the antibody or fragment as defined herein above, in particular the antibody or fragment thereof comprising a human IgM heavy chain constant domain, a human IgG1 heavy chain constant domain, a human IgG2 heavy chain constant domain, a human IgG3 heavy chain constant domain, a human IgG4 heavy chain constant domain, or a human IgA heavy chain constant domain as defined herein above, comprises a human Ig kappa light chain constant domain, or a human Ig lambda light chain constant domain. The immunospecific binding refers to the immunospecific detection and binding of an antibody to an antigenic epitope of a ribosomal protein as defined herein above.

In further embodiments antibodies of the invention include polyclonal, monoclonal, multispecific, human, humanized or chimeric antibodies, whole immunoglobulin molecules, anti-idiotypic (anti-Id) antibodies (including, e. g., anti-Id antibodies to antibodies of the invention).

The term "antibody substructure" as used herein refers to single chain antibodies, Fab fragments, Fab' fragments, fragments produced by a Fab expression library, F(ab')2, Fv, disulfide linked Fv, minibodies, diabodies, scFv, sc(Fv)2, and epitope-binding fragments of any of the above. Preferred are Fab, Fab' and F (ab')2, Fv, single-chain Fvs (scFv), sc(Fv)2, single-chain antibodies, disulfide-linked Fvs (sdFv) and fragments comprising either a VL or VH domain.

The term "Fab fragment" as used herein refers to antibody fragments consisting of the first constant domain of the heavy chain (CH1), the constant domain of the light chain (CL), the variable domain of the heavy chain (VH) and the variable domain of the light chain (VL) of an intact immunoglobulin protein. Typically, Fab fragments are obtained by proteolytic cleavage by papain.

The term "F(ab')2" or "F(ab')2 fragment" as used herein refers to antibody fragments consisting of two first constant domains of the heavy chain (CH1), two constant domains of the light chain (CL), two variable domains of the heavy chain (VH) and two variable domains of the light chain (VL) of an intact immunoglobulin protein, i.e. it comprises two Fab fragments. Additionally, F(ab')2 molecules comprise a S-S linkage in the antibody hinge region wich combines the Fab fragments. Typically, F(ab')2 fragments are obtained by proteolytic cleavage by pepsin.

The term "Fab' fragment" as used herein refers to fragments derived from "F(ab')2" molecules, preferably fragments comprising the S-S linkage in the antibody hinge region.

The term "Fv fragments" as used herein refers to antibody fragments consisting of the two variable antibody domains VH and VL (details may be derived from Skerra and Pluckthun, 1988, Science, 240: 1038-1041).

The term "single chain Fv fragment (scFv)" as used herein relates to antibody fragments consisting of the two VH and VL domains linked together by a flexible peptide linker (details may be derived from Bird and Walker, 1991, Trends Biotechnol., 9: 132-137).

The term "diabody" as used herein refers to an antibody variant comprising a separated VH-VL and VL-VH fusion, wherein the fused domains are linked together by a flexible peptide linker. The linker may have a length of about 1 to 20 amino acids, preferably of between about 2 and 7 amino acids. Typically, small amino acids like glycine may be used for the linker. They may also be combined with other amino acids (details may be derived from Hudson and Kortt, 1999, J. Immunol. Methods, 231(1-2):177-89).

The term "minibody" as used herein refers to a size reduced antibody, e.g. an antibody comprising solely variable domains or lacking constant domains, or comprising the variable heavy domain (details may be derived from Quiocho, 1993, Nature, 362(6418): 293-294).

Antigen-binding antibody fragments, including single-chain antibodies, may comprise the variable region(s) alone or in combination with the entirety or a portion of the hinge region, CH1, CH2, and/or CH3 domains. Also envisaged are antigen-binding fragments comprising any combination of variable region(s) with a hinge region, CH1, CH2, and CH3 domains. The antibodies may be from any animal origin including birds and mammals. Preferably, the antibodies are murine (e. g., mouse and rat), donkey, monkey, rabbit, goat, guinea pig, camel, horse, chicken, or human.

The antibodies according to the present invention are typically monospecific. In certain specific embodiments, the antibody may also be bispecific, or of a greater multispecificity. Multispecific antibodies may be specific for different epitopes of a ribosomal protein as defined herein above, or may be specific for both a ribosomal protein as defined herein above, as well as for a heterologous epitope, such as a heterologous polypeptide or solid support material.

Antibodies of the present invention may be described or specified in terms of the epitope(s) or portion(s) of a polypeptide of the present invention which they recognize or specifically bind. The epitopes may preferably be present on a ribosomal protein as defined herein above, more preferably on Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A,Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, or Homo sapiens ribosomal protein L3.

The term "modified antibody" as used herein refers to derivatives which are modified, for instance by the covalent attachment of any type of molecule to the antibody such that said covalent attachment does not prevent the antibody from specifically binding to the epitope or from generating an anti-idiotypic response. Typical examples of such modifications are glycosylation, acetylation, biotinylation, PEG-ylation, phosphorylation, amidation, derivatization by known protecting/blocking groups, proteolytic cleavage, linkage to a cellular ligand or other protein, etc. Chemical modifications may be carried out by known techniques, including specific chemical cleavage, acetylation, formylation etc. Additionally, the derivative may contain one or more non-classical amino acids.

Antibodies may be produced according to any suitable method known to the person skilled in the art. Polyclonal antibodies may be produced by immunization of animals with the antigen of choice. For example, a ribosomal protein can be administered to various host animals including any eukaryotic, prokaryotic, or phage clone. Monoclonal antibodies of defined specificity may be produced using, for instance, the hybridoma technology developed by Kohler and Milstein (Kohler and Milstein, 1976, Eur. J. Immunol., 6: 511-519). Typically, mice are immunized with a ribosomal protein of the invention or a cell expressing such protein. Once an immune response is detected, e.g., antibodies specific for the antigen are detected in the mouse serum, the mouse spleen is harvested and splenocytes isolated. The splenocytes are then fused by well known techniques to any suitable myeloma cells, for example cells from cell line SP20. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Antibody "fragments" as used herein are antibody sub-structures which recognize specific epitopes and which may be generated by known techniques. For example, Fab and F (ab')2 fragments may be produced by proteolytic cleavage of immunoglobulin molecules, using enzymes such as papain (to produce Fab fragments) or pepsin (to produce F (ab') 2 fragments).

Alternatively, antibodies of the present invention can also be generated using various phage display methods known in the art. In phage display methods, functional antibody domains are displayed on the surface of phage particles, which carry the polynucleotide sequences encoding them. In a particular embodiment, such phage can be utilized to display antigen binding domains expressed from a repertoire or combinatorial antibody library (e. g., human or murine). Phages expressing an antigen binding domain that binds the antigen of interest can be selected or identified with antigen, e. g., using labeled antigen or antigen bound or captured to a solid surface or bead. Phages used in these methods are typically filamentous phages including M13 binding domains expressed from a phage with Fab, Fv or disulfide stabilized Fv antibody domains recombinantly fused to either the phage gene III or gene VIII protein. Examples of phage display methods that can be used to produce antibodies according to the present invention include those disclosed in Brinkman et al., 1995, J. Immunol. Methods 182: 41-50.

Examples of techniques which can be used to produce single-chain Fvs and antibodies include those described in U. S. Patents 4,946,778 and 5,258,498; Huston et al., 1991, Methods in Enzymology 203: 46-88; and Skerra et al., 1988, Science 240: 1038-1040.

An "intrabody" as used herein is an antibody that works within the cell and typically binds to an intracellular protein. The intrabody is preferably modified for intracellular localization. The use of intrabodies may require the expression of the intrabody within the target cell, which can be accomplished, for example, by gene therapy or genetic transformation. In certain embodiments, intrabodies may show special alterations such as a modification of immunoglobulin VL domains for hyperstability, provision of resistance to a reducing intracellular environment, or expression as a fusion protein with maltose binding protein or other stable intracellular proteins. In further embodiments, the intrabody may be a single-chain antibody (scFvs).

In a further embodiment the specifically binding molecule is a non-immunoglobulin protein. The term "non-immunoglobulin protein" refers to a group of highly affine proteins which are capable of specifically binding to target molecules such as ribosomal proteins, but do not comprise immunoglobulin domains or elements. The non-immunoglobulin proteins may offer several distinct mechanisms of binding and preferably have a similar affinity for target structures such as ribosomal proteins as defined herein above as antibodies.

Examples of non-immunoglobulin proteins which are preferably used in the context of the present invention include protein structures comprising ankyrin-repeats. Typically, in designed ankyrin-repeat proteins (DARPins) three, four or preferably five repeat ankyrin motifs are present. These may form a stable protein domain with a large potential target interaction surface. Further details may be derived, for example, from Binz et al., 2003, J. Mol. Biol.; 332(2): 489-503.

A further preferred example of a highly affine non-immunoglobulin protein is an affibody molecule, i.e. a protein based on the Z domain (the immunoglobulin G binding domain) of protein A. In contrast to antibodies, affibody molecules are typically composed of alpha helices and lack disulfide bridges. They may be expressed in soluble and proteolytically stable forms in various host cells. Affibody molecules may further be fused with other proteins. Further details may be derived, for example, from Nord et al., 1997, Nat. Biotechnol.; 15(8): 772-777.

The group of highly affine non-immunoglobulin proteins according to the present invention also comprises adnectins. Adnectins are based on the structure of human fibronectin, in particular its extracellular type III domain, which has a structure similar to antibody variable domains, comprising seven beta sheets forming a barrel and three exposed loops on each side corresponding to the three complementarity determining regions. Adnectins typically lack binding sites for metal ions and central disulfide bonds. They are approximately 15 times smaller than an IgG type antibody and comparable to the size of a single variable domain of an antibody. Adnectins may be customized in order to generate and/or increase specificity for target molecules by modifying the loops between the second and third beta sheets and between the sixth and seventh sheets. Further details may be derived, for example, from Koide and Koide, 2007, Methods Mol. Biol.; 352: 95-109.

A further preferred example is the antibody mimetic anticalin, which is derived from human lipocalin. Anticalins typically have the property of binding protein antigens, as well as small molecule antigens. They are composed of a barrel structure formed by 8 antiparallel beta sheets, connected by loops and an attached alpha helix. Mutagenesis of amino acids at the binding site may allow for changing of affinity and selectivity of the molecule. Further details may be derived, for example, from Skerra, 2008, FEBS J., 275 (11): 2677-83.

Another preferred example is affilin, i.e. a genetically engineered protein with the ability to selectively bind antigens, which is structurally derived from gamma-B crystallin or from ubiquitin. Affilins are typically constructed by modification of near-surface amino acids of gamma-B crystallin or ubiquitin and isolated by display techniques such as phage display. The molecular mass of crystallin and ubiquitin based affilins is typically about one eighth or one sixteenth of an IgG antibody, respectively. This may lead to heat stability up to 90°C and an improved stability towards acids and bases. Further details may be derived, for example, from Ebersbach et al., 2007 J Mol Biol.; 372(1): 172-185 or from Hey et al., 2005, Trends Biotechnol.; 23(10): 514-522.

The group of highly affine non-immunoglobulin proteins also comprises avimers, i.e. artificial proteins that are able to specifically bind to certain antigens via multiple binding sites. Typically, the individual avimer sequences are derived from A domains of various membrane receptors and have a rigid structure, stabilized by disulfide bonds and calcium. Each A domain can bind to a certain epitope of the target molecule. The combination of domains binding to different epitopes of the same target molecule may increases affinity to this target. Further details may be derived, for example, from Silverman et al., 2005, Nat. Biotechnol.; 23(12): 1556-61.

Further preferred examples include knottins, i.e. small disulfide-rich proteins characterized by a special disulfide through disulfide knot. This knot is typically obtained when one disulfide bridge crosses the macrocycle formed by two other disulfides and the interconnecting backbone (disulfide III-VI goes through disulfides I-IV and II-V). Knottin peptides could be shown to bind with high affinity (about 10 to 30 nmol/L) to integrin receptors. The knottin scaffold may accordingly be used for the design of highly affine molecules which are able to bind detection moieties according to the invention. Further details may be derived, for example, from Kimura et al., 2009, Cancer Res., 69; 2435.

The group of highly affine non-immunoglobulin proteins additionally comprises fynomers, i.e. Fyn SH3-derived proteins .Fyn is a 59-kDa member of the Src family of tyrosine kinases. The Fyn SH3 domain comprises 63 residues, and its amino acid sequence is fully conserved among man, mouse, rat, and monkey. Fynomers are typically composed of two antiparallel beta sheets and contain two flexible loops (RT and n-Src loops) to interact with other proteins or targets. Further details may be derived, for example, from Grabulovski et al., 2007, Journal of Biological Chemistry, 282 (5): 3196-3204.

Yet another preferred example of a highly affine non-immunoglobulin molecule is a phylomer peptide. Phylomer peptides are bioactive fragments of naturally occurring proteins that are encoded in the genomes of evolutionary diverse microbes, which are partially sourced from extreme environments and may have evolved over billions of years, providing a multitude of distinct and stable structures capable of binding to biological molecules. Further details may be derived, for example, from Watt, 2009, Future Med. Chem., 1(2): 257-265.

A further, particularly preferred example of a highly affine non-immunoglobulin molecule is an artificial antibody mimic. The term "artificial antibody mimic" as used herein refers to polymeric organic or inorganic structure which specifically binds a target molecule, typically with a binding affinity in the range of an antibody. Examples of such artificial antibody mimics are molecularly imprinted polymers (MIPs). The principle of molecular imprinting relies on the formation of a host-guest complex between a template molecule of interest and one or more functional monomers. The templates allowed to associate with the functional monomers in an appropriate solvent, and a plethora of noncovalent interactions may be formed. In addition, a cross-linking agent may be added, which may provoke subsequent polymerization. Thereby typically a rigid and highly cross-linked polymer matrix is formed which encapsulates the template molecule within. Subsequently, the template molecule may be removed so that an imprint remains, which is complimentary to the original molecule (in particular group of ribosomal proteins, e.g. as present at the surface of a ribosome) in terms of size, shape, and distribution of charge. Molecularly imprinted polymers (MIPs) envisaged by the present invention are acrylamide based polymers, polyacrylamide based polymers, polyethylene based polymers, acrylic acid based polymers, or any derivatives thereof. Examples of derivates are methacrylic acid based polymers or methyl methacrylate based polymers. Further envisaged is any combination or mixture of crylamide based polymers, polyacrylamide based polymers, polyethylene based polymers, acrylic acid based polymers, methacrylic acid based polymers and methyl methacrylate based polymers, or of any other suitable polymer or derivative thereof. Further variations or polymer forms are also envisaged thus included within the scope of the present application. In a further specific embodiment, the MPIs may be used as mold or guidance for the preparation of one or more replica of the MIPs, e.g. by preparing secondary imprints etc. Accordingly, the present invention also envisages the generation and production of 2^{nd}, 3^{rd} or higher level MIPs. Such MIPs may, for example, be produced on a mass production scale. Further information may be available to the skilled person or can be derived from prior art documents such as Withcombe et al., 2011, Chem Soc Rev, 40, 1547-1571.

The group of preferred highly affine non-immunoglobulin molecule also comprises kunitz domain peptides. Kunitz domains are the active domains of Kunitz-type protease inhibitors. They typically have a length of about 50 to 60 amino acids and a molecular weight of 6 kDa. Examples of Kunitz-type protease inhibitors are aprotinin, Alzheimer's amyloid precursor protein (APP), and tissue factor pathway inhibitor (TFPI). Kunitz domains are stable as standalone peptides and are able to recognize specific targets such as protein structure and may accordingly be used for the design of highly affine molecules which are able to bind detection moieties according to the invention. Further details may be derived, for example, from Nixon and Wood, 2006, Curr. Opin. Drug Discov. Devel., 9(2), 261-268.

The term "aptamer" as used herein refers to a nucleic acid and/or peptide that specifically binds to a target, i.e. which binds to a ribosomal protein as defined herein. Aptamers are typically selected from pools of nucleic acids and/or peptides for their selective binding properties.

In specific embodiments the aptamer is a "nucleic acid aptamer", i.e. a short nucleic acid molecule, e.g. RNA, DNA, PNA, CNA, HNA, LNA or ANA or any other suitable nucleic acid format known to the person skilled in the art, being capable of specifically binding to, inter alia, protein target molecules such as a ribosomal protein according to the present invention. The nucleic acid aptamer is typically characterized by binding interaction other than Watson- Crick base pairing or triple helix binding with a second and/or third nucleic acid. Such binding interaction may include Van der Waals interaction, hydrophobic interaction, hydrogen bonding and/or electrostatic interactions. Nucleic acid aptamers are typically generated by in vitro selection or SELEX. These molecules offer potential advantages over antibodies as they can be engineered in a test tube, are easily produced by chemical synthesis and potentially possess desirable storage properties. A nucleic acid aptamer according to the present invention may further be combined with additional moieties, e.g. with interacting portions like biotin or enzymatic functionalities like ribozyme elements.

In a further specific embodiment the aptamer is a "peptide aptamer", i.e. a short peptide capable of interacting and specifically binding to a protein target such as a ribosomal protein as defined herein above. Typically, peptide aptamers comprise a variable peptide loop attached at both ends to a protein scaffold. This double structural constraint is assumed to greatly increase the binding affinity of the peptide aptamer to levels comparable to an antibody's. The variable loop length may preferably be composed of ten to twenty amino acids, while the scaffold may be any protein which has good solubility and compacity properties. For instance, the bacterial protein Thioredoxin-A may be used as scaffold protein. The variable loop may accordingly be inserted within the reducing active site. Alternatively, staphylococcal protein A and domains thereof and derivatives of these domains, such as protein Z, or lipocalins may be used as scaffold structures in the context of the present invention. Peptide aptamers may be generated according to any suitable method known to the person skilled in the art, e.g. by selection from combinatorial peptide libraries, or surface display technologies such as mRNA display, ribosome display, bacterial display and yeast display.

According to a further embodiment of the invention the specifically binding molecule is ribosome binding protein p34. The protein is a leucine-rich repeat protein, which is apparently present as a dimer. The protein p34 was found as non-glycosylated membrane protein characteristic of rough microsomes and is believed to play a role in the ribosome-membrane association. The protein is at least known in humans and rats, with several further homologues in further organisms, which are also envisaged by the present invention. The human ribosome binding protein p34 is deposited with accession no. Q96AG4 in the uniprot database, the rat ribosome binding protein p34 is deposited with accession no. Q5RJR8 in the uniprot database. According to another embodiment of the invention the specifically binding molecule is ER membrane translocator SEC61alpha. The protein is part of the Sec61 complex and apparently a central component of the endoplasmic reticulum (ER) translocation site. The protein is at least known in humans and mice, with several further homologues in further organisms, which are also envisaged by the present invention. The human ER membrane translocator SEC61alpha is deposited with accession no. P61619 in the uniprot database, the mouse ER membrane translocator SEC61alpha is deposited with accession no. P61620 in the uniprot database.

In a further aspect the present invention relates to a method for identifying one or more RNA molecules as defined herein above, which are differentially associated with at least one ribosome, comprising:
(a) contacting a cellular extract as defined herein above, wherein said cellular extract is derived from cells grown under or obtained from conditions selected from the group of: metabolic stress conditions, different nutrient and/or supply conditions, conditions involving the absence or presence of one or more growth factors, differentiation factors, receptor ligands, conditions involving the absence or presence of pharmaceutically active compounds, potentially pharmaceutically active compounds or small molecules, toxic or potentially toxic conditions and/or pathological or potentially pathological conditions as defined herein above, with a molecule specifically binding to a ribosomal protein defined herein above, wherein said specifically binding molecule is immobilized on a substrate, matrix or bead as defined herein above;
(b) contacting a cellular extract as defined herein above, wherein said cellular extract is derived from cells grown under normal, healthy and/or optimal conditions defined herein above, with a molecule specifically binding to a ribosomal protein or group of ribosomal proteins defined herein above, wherein said specifically binding molecule is immobilized on a substrate, matrix or bead defined herein above; and
(c) identifying one or more RNA molecules being directly or indirectly associated with at least one ribosome comprising said ribosomal protein or group of ribosomal proteins which are present under conditions of step (a), but not under conditions of step (b), or one or more RNA molecules which are present under conditions of step (b), but not under conditions of step (a).

The term "differentially associated with at least one ribosome" as used herein refers to the association of an RNA molecule to a ribosome under conditions as outlined in section (a), while not being associated to a ribosome under conditions as outlined in section (b). The term also covers situations in which RNA molecules are associated to a ribosome under conditions as outlined in section (b), while not being associated to a ribosome under conditions as outlined in section (a). The method accordingly identifies RNA molecules which are only associated to the ribosome under one of the above mentioned conditions of one group, but not under those of the other group and vice versa.

In certain embodiments, a differential association may be an at least 2-3 fold difference in the level of association between conditions with less than 3 replicates, e.g. biological replicates. Alternatively, the differential association may also be an association with a p-value < 0.05 with Student's t-test of at least 3 replicates, e.g. biological replicates. The presence of a differential association may further be based on a combination of at least 2-3 fold difference in the level of association between conditions with less than 3 replicates and a p-value < 0.05 with students t-test of at least 3 replicates, e.g. for a more rigid statistic.

This allows to detect RNA molecules which are involved in biological or biochemical process underlying the organisms, tissues or cell's reaction to conditions as mentioned. The identification is implemented by amplification steps and/or sequencing approaches as mentioned herein above. Further envisaged are bioinformatic and data mining procedures, e.g. comparisons with databases, pathway information depositories etc. in order to help interpreting the obtained results.

In a further specific embodiment of the present invention an amplified nucleic acid molecule, e.g. derived from an RNA molecule identified according to the present invention, is introduced into a replicable genetic element. A suitable replicable genetic element according to the present invention may be, for example, a phage, a plasmid, a viral, or a retroviral vector. Retroviral vectors may be replication competent or replication defective. In the latter case, viral propagation generally will occur only in complementing host cells. Amplified nucleic acid molecule according to the present invention may be joined to a vector containing a selectable marker for propagation in a host. Furthermore, the nucleic acid insert may be operatively linked to an appropriate promoter, such as the phage lambda PL promoter, the E. coli lac, trp, phoA and tac promoters, the SV40 early and late promoters and promoters of retroviral LTRs. Other suitable promoters are known to the person skilled in the art. The expression constructs may further contain sites for transcription initiation, termination, and, in the transcribed region, a ribosome binding site for translation. In particular, specific initiation signals may be required for efficient translation of inserted coding sequences. These signals include the ATG initiation codon and adjacent sequences. Furthermore, the initiation codon may typically be in phase with the reading frame of a coding sequence to ensure translation of the entire insert. These exogenous translational control signals and initiation codons can be of a variety of origins, both natural and synthetic. The efficiency of expression may further be enhanced by the inclusion of appropriate transcription enhancer elements etc. In a further specific embodiment the coding portion of the transcripts expressed by the constructs may include a translation initiating codon at the beginning and a termination codon (UAA, UGA or UAG) appropriately positioned at the end of the polypeptide to be translated. The replicable genetic element may include at least one selectable marker. Such markers include, for instance, dihydrofolate reductase, G418, hygromycin or neomycin resistance for eukaryotic cell culture and tetracycline, kanamycin or ampicillin resistance genes for culturing in E. coli and other bacteria. Further selection markers include the herpes simplex virus thymidine kinase, hypoxanthine-guanine phosphoribosyltransferase and adenine phosphoribosyltransferase. Methods commonly known in the art of recombinant DNA technology may be routinely applied to select the desired recombinant clone, and such methods are described, for example, in Ausubel et al., eds, 2007, Current Protocols in Molecular Biology, John Wiley & Sons, Inc., New York. Replicable genetic elements or vectors preferred for use in bacteria include pQE70, pQE60 and pQE9, available from QIAGEN, Inc.; pBluescript vectors, Phagescript vectors, pNH8A, pNH16a, pNH18A, pNH46A, available from Stratagene Cloning Systems, Inc.; pKK223-3, pKK233-3, pDR540, pRIT5 available from Pharmacia Biotech, Inc., and pET vectors available from Novagen. Among preferred eukaryotic replicable genetic elements or vectors are pWLNEO, pSV2CAT, pOG44, pXTI and pSG available from Stratagene; and pSVK3, pBPV, pMSG andpSVL available from Pharmacia. Preferred expression vectors for use in yeast systems include pYES2, pYDI, pTEFI/Zeo, pYES2/GS, pPICZ, pGAPZ, pGAPZalph, pPIC9, pPIC3.5, pHIL-D2, pHIL-SI, pPIC3.5K, pPIC9K, and PAO815. Other suitable replicable genetic elements or vectors are known to the person skilled in the art.

Further described is a method comprising the introduction of the amplified nucleic acid molecule present on the replicable genetic element or vector into a host cell. The term "host cell" as used herein refers to any suitable host cell known to the person skilled in the art. Representative examples of appropriate host cells include bacterial cells, such as E. coli, Streptomyces and Salmonella typhimurium cells; fungal cells, such as yeast cells (e.g., Saccharomyces cerevisiae or Pichia pastoris); insect cells such as Drosophila melanogaster S2 and Spodoptera frugiperda Sf9 cells; mammalian cells, e.g. mouse, rat, monkey or human cell culture cells, and plant cells. In addition, a host cell strain may be chosen which modulates the expression of the inserted sequences, or modifies and processes the gene product in the specific fashion desired. Such modifications (e. g. glycosylation) and processing (e. g. cleavage) of protein products may be important for the function of the protein. Different host cells have characteristic and specific mechanisms for the posttranslational processing and modification of proteins and gene products. Appropriate cell lines or host systems can be chosen to ensure the correct modification and processing of the introduced protein expressed. To this end, eukaryotic host cells, which possess the cellular machinery for proper processing of the primary transcript, glycosylation, and phosphorylation of the gene product are preferred. Such mammalian host cells include HEK 293, Bowes melanoma cells, CHO, VERY, BHK, Hela, COS, MDCK, 293,3T3, W138, and in particular, breast cancer cell lines such as, for example, BT483, Hs578T, HTB2, BT20 and T47D, and normal mammary gland cell line such as, for example, CRL7030 and Hs578Bst. For long-term, high-yield production of recombinant proteins, stable expression is preferred. For example, cell lines which stably express the polypeptide of the present invention may be engineered according to standard procedures known to the person skilled in the art.

The term "introducing the nucleic acid molecule on a replicable genetic element into a host cell" as used herein refers to any suitable cell nucleic acid introduction or transformation technique known to the person skilled in the art. For example, such introduction can be carried out by transfection, e.g. DEAE-dextran mediated transfection, electroporation, microinjection, infection with a viral or bacteriophage vector containing the nucleic acid sequences, cell fusion, chromosome-mediated gene transfer, microcell-mediated gene transfer, cationic lipid-mediated transfection, spheroplast fusion, etc. Further introduction technique contemplated by the present invention include the contacting with defective or attenuated retrovirals, microparticle bombardment, the use of coatings with lipids or cell-surface receptors or transfecting agents, the use of encapsulation in liposomes, microparticles, or microcapsules, for instance by administering them in linkage to a peptide which is known to enter the nucleus, or by administering it in linkage to a ligand subject to receptor-mediated endocytosis. Typically, a plasmid vector is introduced in a precipitate, such as a calcium phosphate precipitate, or in a complex with a charged lipid. If the vector is a virus, it may preferably be packaged in vitro using an appropriate packaging cell line and then transduced into host cells. These and numerous further techniques are known in the art for the introduction of nucleic acid molecules or vectors into cells and may be used in accordance with the present invention. Following the introduction of the nucleic acid, engineered cells may be allowed to grow under suitable conditions as known to the person skilled in the art, e.g. for 1-2 days in an enriched media, and then are switched to a selective media. Appropriate culture media and conditions for the above described host cells and vectors are known in the art.

Also described is a method which further comprises the step of expressing the amplified nucleic acid molecule in the host cell into a polypeptide. This expression step may comprise (a) culturing the recombinant host cell such that the encoded polypeptide is expressed; and optionally (b) recovering said polypeptide. Method for the culturing of recombinant host cells in order to express encoded polypeptides are known in the art. For example, if a regulable promoter is used, an inducing agent may be added to the culture or the appropriate, optimal conditions for working may be set, e.g. a specific temperature, pH, ion concentration etc. The expression of introduced elements may be controlled by numerous standard gene expression tests known to the person skilled in the art. For instance, the transcription of an introduced nucleic acid may be tested in Northern analysis tests and/or the presence of correspondingly translated polypeptides may be tested via Western analysis tests. Further details and additional tests may be derived from qualified textbooks, e.g. from Ausubel et al., eds, 2007, Current Protocols in Molecular Biology, John Wiley & Sons, Inc., New York. The term "recovering" as used herein above refers to any suitable method for the extraction and/or purification of polypeptides from cells, cell suspensions or cell cultures known to the person skilled in the art. Typical recovering methods include ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Preferably, high performance liquid chromatography (HPLC) may be employed for purification.

Also described is a replicable genetic element comprising an amplified nucleic acid obtainable by the method of introducing said amplified nucleic acid into a replicable genetic element. The amplified nucleic acid is derived from an RNA molecule identified and obtained according to the present invention as outlined above. The replicable genetic element may be any suitable replicable genetic element, for example, a phage, a plasmid, a viral, or a retroviral vector. It is preferred that the replicable genetic element is pQE70, pQE60, pQE9 pNH8A, pNH16a, pNH18A, pNH46A, pKK223-3, pKK233-3, pDR540, pRIT5, pWLNEO, pSV2CAT, pOG44, pXTI, pSG, pSVK3, pBPV, pMSG, pSVL, pYES2, pYDI, pTEFI/Zeo, pYES2/GS, pPICZ, pGAPZ, pGAPZalph, pPIC9, pPIC3.5, pHIL-D2, pHIL-SI, pPIC3.5K, pPIC9K, or PAO815 as defined herein above.

Additionally described is a recombinant host cell obtained or obtainable according to the above described introduction techniques. In particular, a host cell comprising a replicable genetic element obtainable by the method of introduction as defined herein above. Such recombinant host cells may contain an amplified nucleic acid molecule derived from an identified RNA molecule or a corresponding vector comprising said molecule according to the present invention. The recombinant host cells may further express a polypeptide encoded by the nucleic acid molecule. Typically, recombinant host cells differ from naturally occurring cells or from known host cells by the presence of additional nucleic acid molecules or fragments thereof, which are not present in natural contexts or in the parental cells or cell lines. For example, recombinant host cells may comprise selection markers, duplicated or multimeric copies of naturally present genes or nucleic acids, heterologous elements like promoter sequences, terminator sequences etc. or genetic identification tags. These elements may be used for the characterization of the recombinant host cells and for their distinguishing from natural contexts and from parental cells or cell lines.

Also described is an expressed polypeptide obtainable by the method of expressing an amplified nucleic acid molecule as defined herein above. Depending upon the host employed in a recombinant production procedure, the accordingly obtained polypeptides or proteins may be glycosylated or may be non-glycosylated. In addition, accordingly obtained polypeptides or proteins may also include an initial modified methionine residue, in some cases as a result of host mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In another aspect the present invention relates to a kit for identifying in a cellular extract one or more RNA molecules, which are directly or indirectly associated with at least one ribosome, comprising a molecule which is specifically binding to a ribosomal protein or a group of ribosomal proteins, wherein said molecule is immobilized on a substrate, matrix or bead; and means for identifying one or more RNA molecules, which are directly or indirectly associated with at least one ribosome comprising said ribosomal protein or group of ribosomal proteins. A molecule which is specifically binding to a ribosomal protein or group of ribosomal proteins as comprised in the kit has been defined herein above. The molecule, e.g. an antibody, intrabody, non-immunoglobulin protein such as a molecularly imprinted polymer (MIP), aptamer or p34/LRRC59 or SEC61alpha is provided in any suitable formulation and format, concentration and amount. The formulation may be chosen for extended shelf-life including stabilizers, suitable buffers and anti degradation agents. Furthermore, the pH of the formulation may be adapted. The kit may further comprise substrate, bead or matrix structures onto which the specifically binding molecule is immobilized, as defined herein above. Preferred are beads that comprise the specifically binding molecule in an immobilized form.

The term "means for identifying one or more RNA molecules" as used herein refers to molecular ingredients which are necessary to arrive at an identification of an RNA molecule as described herein above. The identification may either be carried out by a reverse transcription of the RNA molecule and a subsequent amplification of the amplified product, sequencing on the basis of the reversed transcribed RNA molecule, or sequencing of the RNA molecule directly as defined herein above. The kit may, in dependence of the chosen approach, comprise necessary ingredients in order to perform one, some or essentially all steps of the identification process. For example, the kit may comprise a primer, preferably an oligo-dT primer, nucleotides, a buffer and a reverse transcriptase in order to arrive at a cDNA molecule. The primer or oligonucleotide may be optionally labeled according to methods known in the art, e.g. with labels described herein above. The kit may alternatively comprise a ligase and ligation competent primers and a reverse transcriptase in order to generate and arrive at a cDNA molecule. The kit may further comprise one or more primers and a polymerase allowing for the performance of an amplification. In specific embodiment, the kit may comprise ingredients necessary for the performance of direct RNA sequencing as described herein above, e.g. the DRS method of Helicos. The ingredients of the kit may accordingly be adapted in accordance with the envisaged protocol. The kit may in certain embodiments provide ingredients allowing for an amplification of nucleic acids. The kit may in further embodiments provide ingredients allowing for subsequent sequencing steps or preparation steps for sequencing. Amplified or prepared nucleic acid molecules may subsequently be safeguarded or stored for further analysis in the future or at different locations. Further details would be known to the skilled person or can be derived from suitable literature sources, e.g. documentations of sequencing methods such as those of Helicos, Roche or Applied Biosystem. Furthermore, the kit may comprise an amount of a known nucleic acid molecules or RNA molecules, which can be used for a calibration of the kit or as an internal control. In addition, the kit may comprise accessory ingredients like ions such as bivalent cations or monovalent cations, or hybridization solutions, as well as inhibitors of unwanted enzymatic activity such as an RNase inhibitor etc. Preferred are RNase inhibitors such as RNase guard, RNAsin, RNase OUT™, Superase•IN™ or related RNase inhibitors or heparin. Additionally, the kit may comprise an instruction leaflet and/or may provide information as to the relevance of the obtained results.

In a specific, alternative embodiment of the present invention the kit comprises an RNase activity. The kit correspondingly does not comprise any RNase inhibitor. The RNase activity may be, for example, RNase A, H or P. The presence of RNase is intended to provide a possibility for degrading RNA molecules associated with a ribosome, which are not covered or protected by ribosomal structures. This approach specifically allows to identify those RNA molecule portions which have a direct association with the ribosome. The not directly associated RNA molecules are however degraded and cannot be identified. The kit may be used together with an alternative kit comprising RNase inhibitors thus allowing for a differentiation between directly and indirectly ribosome associated RNA molecules.

The following examples and figures are provided for illustrative purposes. It is thus understood that the examples and figures are not to be construed as limiting. The skilled person in the art will clearly be able to envisage further modifications of the principles laid out herein.

### EXAMPLES

### Example 1

### Mammalian ribosome affinity purification using monoclonal antibodies or related affinity reagents (mRAP)

This Example describes conditions and method steps usable for capturing endogenously formed ribosomes, in particular mammalian ribosomes. Alternative approaches leading to similar results may vary in the amount and/or concentration of reagents. Furthermore, different cellular extracts may be used. These extracts may be adjusted for particular organisms, cell-lines and tissues under study.

### 1. Materials

**Buffer A (=polysome lysis buffer):** 20 mM Tris-HCI (pH 7.5), 5 mM MgCl₂, 300 mM NaCl, 0.5 mM DTT, 1 mg/ml cycloheximide, 0.5 mg/ml heparin, 1% Triton X-100, 10 Units/ml DNAse I (RNase-free; Invitrogen), and protease inhibitor cocktail (e.g. Roche ETDA-free tablets). The buffer is prepared fresh each time and cooled on ice. In case of tissue with high content of RNases 50 U/ml RNase OUT™, 50 U/ml Superase•IN™ or related RNase inhibitor are added to the buffer.

**Antibody binding/ washing buffer:** the antibody binding/ washing buffer to be used is, for example, a buffer as provided with Invitrogen Dynabeads® Protein G Kit. Alternatively, buffer A without DNAse I can be used.

**Bead blocking buffer:** As bead blocking buffer buffer A supplemented with 5% BSA and 0.2 mg/ml heparin is used.

**Buffer B (=wash buffer):** 20 mM Tris-HCI pH 7.5, 140 mM NaCl, 2.5 mM MgCl₂, 0.05% Triton X-100.

**Buffer E (=elution buffer):** 50 mM Tris-HCI (pH 7.5-8), 100 mM NaCl, 20 mM EDTA (pH 8.0). Optionally, 1% SDS is added to the buffer.

### Other components:

- cycloheximide: to obtain a cycloheximide solution 10 mg/ml cycloheximide are dissolved in water and stored at -20°C up to several months. The solution is protected from light
- heparin: to obtain a heparin solution, heparin is dissolved in water to 10 mg/ml and stored at -20°C
- Dynabeads® Protein G (Invitrogen, 100-03D)

### 2. Lysis of mammalian cells

Mammalian cells (e.g. HeLa, HEK293) are grown in suspension. For adherent cells the same solutions are used and the cells are lysed directly on the plate with buffer A. Tissues are homogenized with a mixer mill and glass beads, or with a motor-driven Teflon glass homogenizer.
1. 2-20 x10⁶ cells are grown (at a density of 2x10⁵ cells/ml).
2. Cycloheximide is added to a final concentration of 0.1mg/ml. For collection, cells are spun down in 15 ml Falcon tube at 3,000 g for 4 min.
3. Cells are washed twice with 5 ml ice-cold PBS (phosphate-buffered saline) containing 0.1mg/ml cycloheximide.
4. Cells are resuspended in 1 ml of buffer A. Cell suspension is transferred to a 1.5 ml tube.
5. Cell lysis: the suspension is incubated on ice for 10 min with occasional mixing/ pipetting (via aspiration and expulsion) until the lysate looks uniform without visible cell clumps.
6. The suspension is spun at 12,000 g for 10min. Supernatant is collected and transferred to a 1.5 ml tube.
7. Step 6 is repeated or the lysate is filtrated through a sterile filter.
8. Protein concentration of extract is determined with a commercial kit (e.g. BCA protein assay from Pierce). 2-20 x10⁶ cells typically yield 2-5 mg of protein.

### 3. Coupling of antibodies to protein G/A coupled magnetic beads (Dynabeads).

Protein G coupled beads are used for mouse monoclonal antibodies. Protein A coupled beads are used for rabbit serum or rabbit polyclonal antibodies.

Magnetic beads (Dynabeads), diameter approx. 2-5 µm are used. As an alternative, covalent coupling of antibodies to matrix can be achieved with epoxy-activated Dynabeads (Invitrogen) or other types of carboxylated polybeads (Polysciences Inc.) according to the procedures supplied by the manufacturer.
1. Beads are gently resuspend by pipetting.
2. 25 µl bead suspension is transferred in a 1.5 ml tube
3. The tube is placed on a magnet. The supernatant is removed and beads are resuspended in 400 µL of antibody binding/ washing buffer.
4. The suspension is incubated 5 min at room-temperature (RT), while rotating or placing the tube in a Thermoshaker at 950 rpm (e.g. Thermomixer Compact from Eppendorf).
5. Steps 3 & 4 are repeated twice.
6. The tube is placed on a magnet, the supernatant is removed and the beads are resuspended in 400 µL final volume containing the desired antibody (5-25 µg antibodies). The amount of antibodies may be empirically determined in pilot experiments (e.g. with a rule of thumb: 1 mg of antibodies per ml of beads).
7. The suspension is incubated for 40 min at 4°C while rotating.
8. The tube is placed on a magnet, supernatant is removed and beads are resuspended in 400 µL antibody binding/washing buffer followed by 2x washes with 1ml buffer A.
9. Bead blocking: 1 ml of Buffer A supplemented with 5% BSA and 0.2 mg/ml heparing is added. The suspension is incubated at RT for 30 min while rotating.
10. The tube is placed on a magnet, the supernatant is taken off, and the beads are washed with buffer A.

### 4. Immunopurification of ribosomes from whole cell lysates

1. The lysate is thawed on ice and centrifuged in a microcentrifuge at 14,000g for 10 min at 4°C, then the supernatant is transferred to a new tube on ice.
2. The blocked beads are placed on the magnet and the supernatant is removed.
3. 400 µL of cell lysate (about 1-2.5 mg of total protein) are added to one aliquot of blocked beads and incubated for 1 hour at 4°C in a Thermoshaker at 950 rpm.
4. Tubes are placed on a magnet.
5. Supernatants are transferred to fresh tubes and collected (snap-freeze).
6. 1 ml of buffer B is added, beads are gently resuspended, and shaken for 5 min at 4°C in a Thermomixer at 750 rpm. The tube is placed on a magnet and supernatant is transferred to fresh tubes and snap-frozen (wash sample for control).
7. Step 6 is repeated at least 4 times.
8. Beads are gently resuspended in 1 ml buffer B and suspension is transferred to a fresh 1.5 ml tube (low-bind 1.5 ml tubes from Ambion or Eppendorf may be used).
9. Tubes are placed on a magnet and supernatant is removed.
10. Elution: beads are gently resuspended in 50 µl elution buffer (Buffer E) and incubated in Thermomixer (950 rpm) at 65°C for 5 min.
11. The tube is placed on a magnet and supernatant (=eluate) is removed.
12. Steps 11 and 12 are repeated; subsequently, eluates are combined.

### 5. RNA isolation

Standard procedures are applied to isolate RNA.
- mirVanaTM PARIS™ Kit (Ambion) or total RNA isolation kit from Qiagen are used including the eluation of RNA in 50 µL nuclease-free water.
- Standard phenol/chloroform extraction and precipitation with 75% ethanol/3M sodium acetate are carried out.

### 6. Sequencing

RNA is subjected to next-generation sequencing (Illumina, ABI Solid) using standard protocols.

### Example 2

### Classical Protocol for Polysome Preparation (see Fig. 1)

### 1 Gradient preparation

1.1) 10%, 20%, 30%, 40%, 50% sucrose solutions are prepared in the following buffer:
   Tris-HCl pH 8 20mM
   KCl 140mM
   MgCl₂ 5mM
   DTT 0.5mM
   Cycloheximide 0.1mg/ml
   Heparin 0.5mg/ml
1.2) 2.2ml of each 10%, 20% 30% 40% 50% sucrose are underlayed, starting with the 10%, to make 11ml gradients. They are stored overnight at 4° to allow equilibration.

### 2. Polysome extraction

Extracts are made from 500ml of culture and split to 6 gradients. The volumes in the following protocol are for ∼80ml culture (1 gradient).
2.1) Cells are grown to OD600 0.4-0.6. in YPD
2.2) Cycloheximide is added to a final conc. of 0.1mg/ml, immediately cooled and spun down the cells 6K rpm/4min/4°.
   Extended incubation on ice prior to centrifugation was observed to lead to an increase in the 80S fraction.
2.3) Cells are 2 x resuspended in 2.5ml of fresh lysis buffer and spun as above.
   Lysis buffer is:
   20 mM Tris-CI pH 8.0,
   140 mM KCI,
   1.5 mM MgCI2,
   0.5 mM DTT,
   1% Triton X-100,
   0.1mg/ml cycloheximide,
   1mg/ml Heparin
2.4) Cells are resuspended in 0.7ml lysis buffer, transferred to corex tubes and 2/3 vol. chilled glass beads (0.45-0.55 mm) are added. The suspension is 4 x vortexed hard for 20 sec and cooled on ice for 100 sec.
2.5) The supension is spun at 4.7K rpm (2600g)/5min/4°. The supernatant (∼500ul) is transferred to a 1.6ml tube.
2.6) The suspension is spun at 9.5K rpm/5 min/ 4°. It is transferred to a new 1.6 ml tube.
2.7) It is brought to a final volume of 1 ml and -0.8 ml are loaded per gradient.
2.8) Cells are spun at 35K/ 160min/ 4° in SW41 rotor.

### 3. Fractions collection

Gradients are fractionated using ISCO collection system with the following setting:
Pump speed 0.75ml/min
Fraction time 1.2 min/fraction
Chart speed 60cm/hr.
These settings result in 14 fractions with volume of ∼0.9ml. Sensitivity of the OD254 recorder is set to 1 or 2, depending on the desired resolution. Fractions are collected into tubes containing 2ml 8M guanidinium-HCI (final guanidinium concentration is 5.5M).

### 4. RNA extraction for microarray/ sequencing analysis

To obtain sufficient amounts of mRNA for the microarray analysis identical fractions from at least 3 gradients are pooled together.
Indicated volumes are for material from 3 gradients. 75% of the resulting RNA is used for labeling.
4.1) Similar fraction are pooled and precipitated by adding equal volume of 100% Ethanol and incubated overnight at -20°.
4.2) Fractions are spun at 10K/20 min/ 4° using a SS34 rotor. They are subsequently washed with 1ml 85% ethanol and spun as above.
4.3) They are resuspended in 400ul TE pH8, transferred to a 1.6 ml tube and precipitated again by adding 0.1 vol. 3M NaAcetate pH5.3 and 2.5 vol. 100% ethanol.
4.4) Subsequently, they are resuspended in 650 µl DDW and any residual proteins are removed by extracting once with Tris buffered Phenol:Chloroform. 500µl of the aqueous phase are taken to a new tube. '
4.5) The suspension is brought to 1ml with DDW, and LiCI (1.5M final conc.) is added to remove any residual Heparin. The suspension is incubated overnight in -20°. The solution is spun down, washed with 75% ethanol, air dried and resuspended in 155µl.
4.6) To remove the LiCI, it is precipitated again with 0.1 vol. 3M Na Acetate pH5.3 and 3 vol. 100% Ethanol, washed with 75% Ethanol and air dried.
4.7) Cells are resuspended in 1mM Tris pH 7.4 (20µl for material from 3 gradients, typically 10-15µl from this are used for labeling).

### Example 3

### Immunoblot analysis with RPL antibodies

1) A 12% SDS-PAGE gel is run. Therefore, 10-20 µg of cell-free extract is loaded per lane.
2) Proteins are transferred onto nitrocellulose membrane by wet-transfer (BioRad; 1 hour, 100mA). The membrane is rinsed in distilled H₂O and stained with Ponceau-S solution (Sigma). *The lanes with extract should give clear staining which is comparable to Marker lane.*
3) Membranes are blocked in PBST (1xPBS, 0.1% Tween-20), 5% BSA over night (8-12 hours) at 4°C on a shaker.
4) The membrane is incubated with fresh 25 ml PBST/ 2.5% BSA and desired monoclonal/polyclonal **anti-RPL antibody** is added (1:400 - 1:2,000 depending on antibody). Membrane is incubated on a shaker for 90 min at RT.
5) The membrane is washed
   3 x for 15 min with PBST at RT.
   1 x for 30 min in PBST/ 2.5% BSA at RT
6) HRP-coupled secondary antibody is coupled for chemiluminescence detection, for instance a donkey anti-mouse HRP (Amersham; 1:5,000) in PBST/ 2.5% BSA for one hour at RT.
7) The membrane is washed with:
   1x 30 min in PBST/ 2.55% BSA at RT; and
   3x 15 min in PBST at RT.
8) Detection is carried out by developing using an ECL-Kit (Amersham).

### Example 4

### Immunoprecipitation (IP) of the ribosomal proteins and associated mRNA from HEK293 cell lysates using rabbit-anti-RPL26 monoclonal antibodies

HEK293 cells were grown to 75% confluency (approximately 15x10⁶ cells in a 15 cm² dish) and treated with 0.1 mg/ml cycloheximide at 37°C for 5 min. Cells were harvested by gentle scraping of cells from the plate and collected by centrifugation and washed twice with PBS (see Example 1). Cells were lysed in 500 µl of lysis buffer (20 mM Tris-HCI pH 7.5, 150 mM NaCl, 5 mM MgCl₂, 0.1% Triton X-100, 0.5 mg/ml heparin, 0.1 mg/ml cycloheximide, 0.5 mM DTT, 50 U/ml RNase inhibitor, 10 U/ml DNase I, 1X protease inhibitor cocktail [Roche]) at 4°C for 10 min. Cell lysate was centrifuged to pellet nuclei at 4°C for 10 min and 10,000 g. Protein concentration of extracts was determined with the BCA assay kit (Pierce) and 250 µg was used per immunoprecipitation reaction.

Epoxy-coupled beads were prepared as per manufacturers instructions (Life Technologies Novex, Dynabeads antibody coupling kit). Thereby, 25 µl of magnetic beads were coupled to 5 µg rabbit monoclonal RPL26 antibodies or rabbit IgG per reaction. The beads were then added to the extract and the volume was bought to 100 µl with lysis buffer. The reaction was incubation for several hours at 4°C with gentle rotation. Beads were captured by a magnet and further washed three times in 100µl lysis buffer for 5 min under gentle rotation. Finally, RNA and protein was eluted in 100 µl SDS buffer (50 mM Tris pH8.0, 100 mM NaCl, 10 mM EDTA, 1% SDS) by incubation at 65°C for 10 min with gentle agitation. RNA was isolated with the RNA MicroPrep kit (Zymo Research) as per the manufacturer's instructions, including on-column DNase-treatment. A sample of the eluate was boiled in SDS-PAGE loading buffer and run on a 12% polyacrylamide gel and stained with silver (see Fig. 5A). Immunoblot analysis of samples was performed according to protocols outlined in Example 3 (see Fig. 5B). Semiquantitative reverse-transcription PCR was carried out according to standard protocols with 100 ng of RNA applying a 1:1 mixture of oligo-dT and random nonamer primers for RT, and gene specific primers for PCR (see Fig. 5C).

### Semiquantitative reverse-transcription PCR

The semiquantitative reverse-transcription PCR was performed according to the following protocol. The protocol was designed to test for the presence of specific transcripts in affinity isolates of RNA-binding proteins/ ribosomes.

### 1. RNA

1 µg of total RNA was obtained from extract. It was essential to treat with DNAse prior to RT reaction (e.g. Turbo DNA-free Kit from Ambion). 100 ng of immunopurified RNA and equal volumes of respective controls were used.

### 2. Reverse transcription

1) RNA was resuspended in 15 µl primer mix (in 0.2ml PCR tubes):

| **Priming reaction** | **Concentration** | **µl** |
|---|---|---|
| Oligo(dT)/pdN₉ mix | 5µg/µl each | 2 · 1 |
| total RNA (1µg) or IP RNA (100ng) | Measured with NanoDrop | x |
| H₂O | | 13-x |
| *Total* | | *15* |

The mixture was incubated for 10 min at 70°C (PCR machine) >> 5 min on ice.
2) 15 µl of RT-Mix were added (total volume 30 µl).

| **Cocktail** | **µl** |
|---|---|
| 5x RT buffer | 6 |
| 0.1M DTT | 3 |
| 10mM dNTPs | 1.5 |
| RNAseOUT/RNAsin | 0.5 |
| H₂O | 4 |

3) 10 µl were removed into a second tube for mock incubation (Reaction B)
4) 0.5 µl of RT II/ MMLV (200U/ µl) were added to the remaining 20 µl (RT-reaction)
5) The mixture was incubated for 2 h at 42°C (in PCR machine) >> 5 min 95°C >> 5 min on ice.

### 3. PCR

1) A PCR premix was prepared for 25 µl PCR reactions (0.2 ml PCR tubes)

| **Cocktail** | **µl** |
|---|---|
| GO taq buffer (green) | 5 |
| 10mM dNTPs | 0.5 |
| Oligo Fwd&Rev (50pMol/ µl each) | 1 |
| H₂O | 16.5 |
| GO Taq (Promega) | 0.5 |

23.5 µl of PCR premix were added to 1.5 µl of the RT reactions from above.
2) PCR conditions:
3 min 95C >> 28x (95°C 30sec; 59°C 30sec; 72°C 1min) >> 72°C 2min >> 4°C
3) 10 µl of the PCR reaction were visualized on a 1% TAE-agarose gel.

Primers for PCR (Sigma):
p53 Fwd, 5'-ACTGGGACGGAACAGCTTTG-3' (SEQ ID NO: 1);
p53 Rev, 5'-AGAGGAGCTGGTGTTGTTGG-3' (SEQ ID NO: 2);
NFkB-p105 Fwd, 5'-TGCACTGTAACTGCTGGACC-3' (SEQ ID NO: 3);
NFkB-p105 Rev, 5'-AATAGGCAAGGTCAGGGTGC-3' (SEQ ID NO: 4);
GAPDH Fwd, 5'-GGCTCTCCAGAACATCATCC-3' (SEQ ID NO: 5);
GAPDH Rev, 5'-TTTCTAGACGGCAGGTCAGG-3' (SEQ ID NO: 6);
RNU6 Fw, 5'-CGCTTCGGCAGCACATATAC-3 (SEQ ID NO: 7); and
RNU6 Rev, 5'-GGAACGCTTCACGAATTTGC-3' (SEQ ID NO: 8).

### Example 5

### MS analysis

An MS analysis of the sample shown in Fig. 5A was performed. The results are provided in Fig. 6. The diagram on top shows composition of the analysis by (%); the table below shows the identity of ribosomal proteins detected in the sample.

In total, 23 ribosomal proteins were identified (entire ribosomes has 79 proteins). In addition, proteins that may be associated with ribosomes were identified.

## Claims

1. A method for identifying one or more RNA molecules, which are directly or indirectly associated with at least one ribosome, comprising:
(a) contacting a cellular extract with a molecule specifically binding to a ribosomal protein or a group of ribosomal proteins, wherein said specifically binding molecule is immobilized on a substrate, matrix or bead; and
(b) identifying one or more RNA molecules, which are directly or indirectly associated with at least one ribosome comprising said ribosomal protein or group of ribosomal proteins.

2. The method of claim 1, wherein said RNA molecule is an mRNA or non-coding RNA molecule, which is directly associated with at least one ribosome; or a non-coding RNA molecule, which is indirectly associated with at least one ribosome, wherein said non-coding RNA molecule is preferably a microRNA molecule, long ncRNA molecule, short ncRNA molecule, siRNA molecule, antisense RNA molecule, or a regulatory RNA molecule.

3. The method of claim 2, wherein essentially all or all mRNA or non-coding RNA molecules which are directly associated with at least one ribosome are identified, or wherein essentially all or all non coding RNA molecules which are indirectly associated with at least one ribosome are identified, or wherein said indirect association with at least one ribosome comprises a direct association of said non coding RNA molecule with said mRNA molecule, wherein said mRNA molecule is directly associated with at least one ribosome.

4. The method of any one of claims 1 to 3, wherein said identification of one or more RNA molecules comprises an amplification of the RNA molecule to a nucleic acid molecule, wherein said identification of one or more RNA molecules preferably comprises a determination of the sequence of the RNA molecule, preferably by sequencing of reverse transcribed complementary DNA or direct RNA sequencing.

5. The method of any one of claims 1 to 4, wherein said specifically binding molecule is an antibody, intrabody, antibody substructure, or modified antibody, or a fragment thereof, or a non-immunoglobulin protein, preferably selected from the groups of:
(i) designed ankyrin-repeat protein (DARPin), affibody molecule, adnectin, anticalin, affilin, avimer, knottin, fynomer, phylomer, kunitz domain peptide and an artificial antibody mimic such as a molecularly imprinted polymer (MIP), wherein said molecularly imprinted polymer (MIP) is preferably an acrylamide based polymer, a polyethylene based polymer, an acrylic acid based polymer, or a derivative thereof such as a methacrylic acid based polymer or a methyl methacrylate based polymers, or any combination or mixture thereof; or
(ii) an aptamer, more preferably a nucleic acid aptamer or a peptide aptamer; or
(iii) ribosome binding protein p34 (LRRC59), or ER membrane translocator SEC61alpha, or a fragment thereof.

6. The method of any one of claims 1 to 5, wherein said ribosomal protein is a eukaryotic ribosomal protein or a prokaryotic or archaea homolog thereof, wherein said eukaryotic ribosomal protein is preferably a mammalian ribosomal protein, more preferably at least one ribosomal protein selected from the group of: Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A, Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, and Homo sapiens ribosomal protein L38, or a homologue thereof of a eukaryotic, prokaryotic or archaea species.

7. The method of any one of claims 1 to 6, wherein said cellular extract is an extract derived from a prokaryotic cell, an archaebacterial cell, a eukaryotic cell, a mitochondrium, or the cytoplasm of a eukaryotic cell, wherein said mitochondrium is preferably a mitochondrium of a mammalian cell, more preferably a mitochondrium of a human cell, or wherein said cellular extract is a tissue extract derived from a eukaryotic organism, preferably a mammalian tissue extract, more preferably a human tissue extract.

8. The method of any one of claims 1 to 7, wherein said cellular or tissue extract is derived from cells or tissues grown under or obtained from conditions selected from the group of: metabolic stress conditions, specific nutrient and/or supply conditions, conditions involving the absence or presence of one or more growth factors, differentiation factors, or receptor ligands, conditions involving the absence or presence of pharmaceutically active compounds, potentially pharmaceutically active compounds or small molecules, toxic or potentially toxic conditions, pathological or potentially pathological conditions; and normal, healthy or optimal conditions, , wherein said method preferably comprises the identification of one or more RNA molecules associated with at least one ribosome under two or more of conditions selected from the group of: metabolic stress conditions, specific nutrient and/or supply conditions, conditions involving the absence or presence of one or more growth factors, differentiation factors, or receptor ligands, conditions involving the absence or presence of pharmaceutically active compounds, potentially pharmaceutically active compounds or small molecules, toxic or potentially toxic conditions and/or pathological or potentially pathological conditions and normal, healthy or optimal conditions, preferably including a comparison of said identified RNA molecules obtained under two or more of said conditions.

9. A method for identifying one or more RNA molecules, which are differentially associated with at least one ribosome, comprising:
(a) contacting a cellular extract, wherein said cellular extract is derived from cells grown under or obtained from conditions selected from the group of: metabolic stress conditions, different nutrient and/or supply conditions, conditions involving the absence or presence of one or more growth factors, differentiation factors, receptor ligands, conditions involving the absence or presence of pharmaceutically active compounds, potentially pharmaceutically active compounds or small molecules, toxic or potentially toxic conditions and/or pathological or potentially pathological conditions, with a molecule specifically binding to a ribosomal protein or a group of ribosomal proteins, wherein said specifically binding molecule is immobilized on a substrate, matrix or bead;
(b) contacting a cellular extract, wherein said cellular extract is derived from cells grown under normal, healthy and/or optimal conditions, with a molecule specifically binding to a ribosomal protein or a group of ribosomal proteins, wherein said specifically binding molecule is immobilized on a substrate, matrix or bead; and
(c) identifying one or more RNA molecules being directly or indirectly associated with at least one ribosome comprising said ribosomal protein or group of ribosomal proteins which are present under conditions of step (a), but not under conditions of step (b), or one or more RNA molecules which are present under conditions of step (b), but not under conditions of step (a).

10. The method of claim 9, wherein said RNA molecule is an mRNA or non-coding RNA molecule which is directly associated with at least one ribosome or a non-coding RNA molecule, which is indirectly associated with at least one ribosome, wherein said non-coding RNA molecule is preferably a microRNA molecule, long ncRNA molecule, short ncRNA molecule, siRNA molecule, antisense RNA molecule, or a regulatory RNA molecule.

11. The method of claim 9 or 10, wherein said identification of one or more RNA molecules comprises an amplification of the RNA molecule to a nucleic acid molecule or a determination of the sequence of the RNA molecule, preferably by sequencing of reverse transcribed complementary DNA or direct RNA sequencing.

12. The method of any one of claims 9 to 11, wherein said specifically binding molecule is an antibody, intrabody, antibody substructure, or modified antibody, or a fragment thereof, or a non-immunoglobulin protein, wherein said non-immunoglobulin protein is preferably selected from the groups of:
(i) designed ankyrin-repeat protein (DARPin), affibody molecule, adnectin, anticalin, affilin, avimer, knottin, fynomer, phylomer, kunitz domain peptide and an artificial antibody mimic such as a molecularly imprinted polymer (MIP), wherein said molecularly imprinted polymer (MIP) is preferably an acrylamide based polymer, a polyethylene based polymer, an acrylic acid based polymer, or a derivative thereof such as a methacrylic acid based polymer or a methyl methacrylate based polymers, or any combination or mixture thereof; or
(ii) an aptamer, more preferably a nucleic acid aptamer or a peptide aptamer; or
(iii) ribosome binding protein p34 (LRRC59), or ER membrane translocator SEC61alpha, or a fragment thereof.

13. A kit for identifying in a cellular extract one or more RNA molecules, which are directly or indirectly associated with at least one ribosome, comprising:
(i) a molecule which is specifically binding to a ribosomal protein or a group of ribosomal proteins, wherein said molecule is immobilized on a substrate, matrix or bead; and
(ii) means for identifying one or more RNA molecules, which are directly or indirectly associated with at least one ribosome comprising said ribosomal protein or group of ribosomal proteins, wherein said RNA molecule is preferably an mRNA or non-coding RNA molecule which is directly associated with at least one ribosome or a non-coding RNA molecule, which is indirectly associated with at least one ribosome, and wherein said non-coding RNA molecule is preferably a microRNA molecule, long ncRNA molecule, short ncRNA molecule, siRNA molecule, antisense RNA molecule, or a regulatory RNA molecule.

14. The kit of claim 13, wherein said specifically binding molecule is a non-immunoglobulin protein, preferably selected from the groups of:
(i) designed ankyrin-repeat protein (DARPin), affibody molecule, adnectin, anticalin, affilin, avimer, knottin, fynomer, phylomer, kunitz domain peptide and an artificial antibody mimic such as a molecularly imprinted polymer (MIP), wherein said molecularly imprinted polymer (MIP) is preferably an acrylamide based polymer, a polyethylene based polymer, an acrylic acid based polymer, or a derivative thereof such as a methacrylic acid based polymer or a methyl methacrylate based polymers, or any combination or mixture thereof; or
(ii) an aptamer, more preferably a nucleic acid aptamer or a peptide aptamer; or
(iii) ribosome binding protein p34 (LRRC59), or ER membrane translocator SEC61alpha, or a fragment thereof.

15. The kit of claim 13 or 14, wherein said ribosomal protein is a mammalian ribosomal protein, preferably at least one ribosomal protein selected from the group of: Homo sapiens ribosomal protein L3, Homo sapiens ribosomal protein L4, Homo sapiens ribosomal protein L5, Homo sapiens ribosomal protein L6, Homo sapiens ribosomal protein L7, Homo sapiens ribosomal protein L7A, Homo sapiens ribosomal protein L9, Homo sapiens ribosomal protein L10, Homo sapiens ribosomal protein L11, Homo sapiens ribosomal protein L12, Homo sapiens ribosomal protein L13, Homo sapiens ribosomal protein L13A, Homo sapiens ribosomal protein L14, Homo sapiens ribosomal protein L15, Homo sapiens ribosomal protein L17, Homo sapiens ribosomal protein L18, Homo sapiens ribosomal protein L18A, Homo sapiens ribosomal protein L19, Homo sapiens ribosomal protein L22, Homo sapiens ribosomal protein L23A, Homo sapiens ribosomal protein L26, Homo sapiens ribosomal protein L27A, Homo sapiens ribosomal protein L28, Homo sapiens ribosomal protein L29, Homo sapiens ribosomal protein L31, Homo sapiens ribosomal protein L32, Homo sapiens ribosomal protein L35, Homo sapiens ribosomal protein L35A Homo sapiens ribosomal protein L37, and Homo sapiens ribosomal protein L38, or a homologue thereof of a eukaryotic, prokaryotic or archaea species.

## Patentansprüche

1. Verfahren zum Identifizieren eines RNA-Moleküls oder mehrerer RNA-Moleküle, welche/s direkt oder indirekt assoziiert ist/sind mit wenigstens einem Ribosom, umfassend:
(a) Inkontaktbringen eines zellulären Extrakts mit einem Molekül, welches spezifisch an ein ribosomales Protein oder eine Gruppe von ribosomalen Proteinen bindet, wobei das spezifisch bindende Molekül auf einem Substrat, einer Matrix oder einer Perle immobilisiert ist, und
(b) Identifizieren eines RNA-Moleküls oder mehrerer RNA-Moleküle, welche/s direkt oder indirekt assoziiert ist/sind mit wenigstens einem Ribosom, umfassend das ribosomale Protein oder die Gruppen von ribosomalen Proteinen.

2. Verfahren gemäß Anspruch 1, wobei das RNA-Molekül ein mRNA- oder nicht-codierendes RNA-Molekül, welches direkt assoziiert ist mit wenigstens einem Ribosom, oder ein nicht-codierendes RNA-Molekül, welches indirekt assoziiert ist mit wenigstens einem Ribosom, ist, wobei das nicht-codierende RNA-Molekül vorzugsweise ein Mikro-RNA-Molekül, ein langes ncRNA-Molekül, ein kurzes ncRNA-Molekül, ein siRNA-Molekül, ein Antisense-RNA-Molekül oder ein regulatorisches RNA-Molekül ist.

3. Verfahren gemäß Anspruch 2, wobei im Wesentlichen alle oder alle mRNA- oder nicht-codierenden RNA-Moleküle, welche direkt assoziiert sind mit wenigstens einem Ribosom, identifiziert werden, oder wobei im Wesentlichen alle oder alle nicht-codierenden RNA-Moleküle, welche indirekt assoziiert sind mit wenigstens einem Ribosom, identifiziert werden, oder wobei die indirekte Assoziation mit wenigstens einem Ribosom eine direkte Assoziation des nicht-codierenden RNA-Moleküls mit dem mRNA-Molekül umfasst, wobei das mRNA-Molekül direkt assoziiert ist mit wenigstens einem Ribosom.

4. Verfahren gemäß einem der Ansprüche 1 bis 3 wobei die Identifikation von einem oder mehreren RNA-Molekül(en) eine Amplifikation des RNA-Moleküls zu einem Nucleinsäuremolekül umfasst, wobei die Identifikation von einem oder mehreren RNA-Molekül(en) vorzugsweise eine Bestimmung der Sequenz des RNA-Moleküls, vorzugsweise durch Sequenzierung von revers transkribierter komplementärer DNA oder durch direkte RNA-Sequenzierung, umfasst.

5. Verfahren gemäß einem der Ansprüche 1 bis 4, wobei das spezifisch bindende Molekül ein Antikörper, ein Intrabody, eine Antikörpersubstruktur, oder ein modifizierter Antikörper oder ein Fragment davon, oder ein Nicht-Immunglobulin-Protein ist, vorzugsweise ausgewählt aus den Gruppen aus:
(i) Designed-Ankyrin-Repeat-Protein (DARPin), Affibody-Molekül, Adnectin, Anticalin, Affilin, Avimer, Knottin, Fynomer, Phylomer, Kunitz-Domänenpeptid und einem künstlichen Antikörpermimetikum, wie z.B. einem molekulargeprägten Polymer (MIP), wobei das molekulargeprägte Polymer (MIP) vorzugsweise ein Acrylamid-basiertes Polymer, ein Polyethylen-basiertes Polymer, ein Acrylsäurebasiertes Polymer oder ein Derivat davon, wie z.B. ein Methacrylsäure-basiertes Polymer oder ein Methylmethacrylat-basiertes Polymer, oder jede Kombination oder Mischung davon ist, oder
(ii) einem Aptamer, noch bevorzugter einem Nucleinsäureaptamer oder einem Peptidaptamer, oder
(iii) Ribosomenbindungsprotein p34 (LRRC59) oder ER-Membrantranslokator SEC61alpha oder einem Fragment davon.

6. Verfahren gemäß einem der Ansprüche 1 bis 5, wobei das ribosomale Protein ein eukaryotisches ribosomales Protein oder ein prokaryotisches oder Archaeenhomolog davon ist, wobei das eukaryotische ribosomale Protein vorzugsweise ein ribosomales Säugetierprotein, noch bevorzugter wenigstens ein ribosomales Protein, ausgewählt aus der Gruppe aus: ribosomalem Homo sapiens Protein L3, ribosomalem Homo sapiens Protein L4, ribosomalem Homo sapiens Protein L5, ribosomalem Homo sapiens Protein L6, ribosomalem Homo sapiens Protein L7, ribosomalem Homo sapiens Protein L7A, ribosomalem Homo sapiens Protein L9, ribosomalem Homo sapiens Protein L10, ribosomalem Homo sapiens Protein L11, ribosomalem Homo sapiens Protein L12, ribosomalem Homo sapiens Protein L13, ribosomalem Homo sapiens Protein L13A, ribosomalem Homo sapiens Protein L14, ribosomalem Homo sapiens Protein L15, ribosomalem Homo sapiens Protein L17, ribosomalem Homo sapiens Protein L18, ribosomalem Homo sapiens Protein L18A, ribosomalem Homo sapiens Protein L19, ribosomalem Homo sapiens Protein L22, ribosomalem Homo sapiens Protein L23A, ribosomalem Homo sapiens Protein L26, ribosomalem Homo sapiens Protein L27A, ribosomalem Homo sapiens Protein L28, ribosomalem Homo sapiens Protein L29, ribosomalem Homo sapiens Protein L31, ribosomalem Homo sapiens Protein L32, ribosomalem Homo sapiens Protein L35, ribosomalem Homo sapiens Protein L35A ribosomalem Homo sapiens Protein L37 und ribosomalem Homo sapiens Protein L38, oder einem Homolog davon oder aus einer eukaryotischen, prokaryotischen oder Archaeenspezies, ist.

7. Verfahren gemäß einem der Ansprüche 1 bis 6, wobei der zelluläre Extrakt ein Extrakt ist, der aus einer prokaryotischen Zelle, einer archaeaenbakteriellen Zelle, einer eukaryotischen Zelle, einem Mitochondrium, oder dem Zytoplasma einer eukaryotischen Zelle stammt, wobei das Mitochondrium vorzugsweise ein Mitochondrium einer Säugetierzelle, noch bevorzugter ein Mitochondrium aus einer humanen Zelle ist, oder wobei der zelluläre Extrakt ein Gewebeextrakt ist, der aus einem eukaryotischen Organismus stammt, vorzugsweise ein Säugetiergewebeextrakt ist, noch bevorzugter ein humaner Gewebeextrakt ist.

8. Verfahren gemäß einem der Ansprüche 1 bis 7, wobei der zelluläre oder Gewebeextrakt aus Zellen oder Geweben stammt, die unter Bedingungen kultiviert oder erhalten werden, die ausgewählt sind aus der Gruppe aus: metabolischen Stressbedingungen, spezifischen Nährstoff- und/oder Zuführbedingungen, Bedingungen, bei denen ein oder mehrere Wachstumsfaktor(en), Differenzierungsfaktor(en) oder Rezeptorligand(en) fehlt/fehlen oder vorhanden ist/sind, Bedingungen, bei denen pharmazeutisch wirksame Verbindungen, potenziell pharmazeutisch wirksame Verbindungen oder kleine Moleküle, fehlen oder vorhanden sind, toxischen oder potenziell toxischen Bedingungen, pathologischen oder potenziell pathologischen Bedingungen und normalen, gesunden oder optimalen Bedingungen, wobei das Verfahren vorzugsweise die Identifikation von einem oder mehreren RNA-Molekül(en), welche/s assoziiert ist/sind mit wenigstens einem Ribosom bei zwei oder mehr Bedingungen, ausgewählt aus der Gruppe aus: metabolischen Stressbedingungen, spezifischen Nährstoff- und/oder Zuführbedingungen, Bedingungen, bei denen ein oder mehrere Wachstumsfaktor(en), Differenzierungsfaktor(en) oder Rezeptorligand(en) fehlt/fehlen oder vorhanden ist/sind, Bedingungen, bei denen pharmazeutisch wirksame Verbindungen, potenziell pharmazeutisch wirksame Bedingungen oder kleine Moleküle, fehlen oder vorhanden sind, toxischen oder potenziell toxischen Bedingungen, pathologischen oder potenziell pathologischen Bedingungen, und normalen, gesunden oder optimalen Bedingungen, vorzugsweise einschließlich eines Vergleichs der identifizierten RNA-Moleküle, die bei zwei oder mehr der Bedingungen erhalten wurden, umfasst.

9. Verfahren zum Identifizieren von einem RNA-Molekül oder mehreren RNA-Molekülen, welche/s differenziell mit mindestens einem Ribosom assoziiert ist/sind, umfassend:
(a) Inkontaktbringen eines zellulären Extrakts, wobei der zelluläre Extrakt aus Zellen stammt, die unter Bedingungen kultiviert oder erhalten werden, die ausgewählt sind aus der Gruppe aus: metabolischen Stressbedingungen, unterschiedlichen Nährstoff- und/oder Zuführbedingungen, Bedingungen, bei denen ein oder mehrere Wachstumsfaktor(en), Differenzierungsfaktor(en) oder Rezeptorligand(en) fehlt/fehlen oder vorhanden ist/sind, Bedingungen, bei denen pharmazeutisch wirksame Verbindungen, potenziell pharmazeutisch wirksame Verbindungen oder kleine Moleküle, fehlen oder vorhanden sind, toxischen oder potenziell toxischen Bedingungen, und/oder pathologischen oder potenziell pathologischen Bedingungen, mit einem Molekül, das spezifisch an ein ribosomales Protein oder eine Gruppe von ribosomalen Proteinen bindet, wobei das spezifische Molekül auf einem Substrat, einer Matrix oder einer Perle immobilisiert ist,
(b) Inkontaktbringen eines zellulären Extrakts, wobei der zelluläre Extrakt aus Zellen stammt, die unter normalen, gesunden und/oder optimalen Bedingungen kultiviert wurden, mit einem Molekül, das spezifisch an ein ribosomales Protein oder eine Gruppe von ribosomalen Proteinen bindet, wobei das spezifisch bindende Molekül auf einem Substrat, einer Matrix oder einer Perle immobilisiert ist, und
(c) Identifizieren eines RNA-Moleküls oder mehrerer RNA-Moleküle, welche/s direkt oder indirekt mit wenigstens einem Ribosom assoziiert ist/sind, umfassend das ribosomale Protein oder die Gruppe von ribosomalen Proteinen, welche bei Bedingungen von Schritt (a) anwesend sind, jedoch nicht bei Bedingungen von Schritt (b), oder eines RNA-Moleküls oder mehrerer RNA-Moleküle, welche/s bei Bedingungen von Schritt (b) vorhanden ist/sind, jedoch nicht bei Bedingungen von Schritt (a).

10. Verfahren gemäß Anspruch 9, wobei das RNA-Molekül ein mRNA- oder nicht-codierendes RNA-Molekül, welches direkt assoziiert ist mit wenigstens einem Ribosom, oder ein nicht-codierendes RNA-Molekül, welches indirekt assoziiert ist mit wenigstens einem Ribosom ist, wobei das nicht-codierende RNA-Molekül vorzugsweise ein Mikro-RNA-Molekül, ein langes ncRNA-Molekül, ein kurzes ncRNA-Molekül, ein siRNA-Molekül, ein Antisense-RNA-Molekül oder ein regulatorisches RNA-Molekül ist.

11. Verfahren gemäß Anspruch 9 oder 10, wobei die Identifikation von einem oder mehreren RNA-Molekül(en) eine Amplifikation des RNA-Moleküls zu einem Nucleinsäuremolekül oder eine Bestimmung der Sequenz des RNA-Moleküls, vorzugsweise durch Sequenzierung von revers transkribierter komplementärer DNA oder durch direkte RNA-Sequenzierung, umfasst.

12. Verfahren gemäß einem der Ansprüche 9 bis 11, wobei das spezifisch bindende Molekül ein Antikörper, ein Intrabody, eine Antikörpersubstruktur oder ein modifizierter Antikörper oder ein Fragment davon, oder ein Nicht-Immunglobulin-Protein ist, wobei das Nicht-Immunglobulin-Protein vorzugsweise ausgewählt ist aus den Gruppen aus:
(i) Designed-Ankyrin-Repeat-Protein (DARPin), Affibody-Molekül, Adnectin, Anticalin, Affilin, Avimer, Knottin, Fynomer, Phylomer, Kunitz-Domänenpeptid und einem künstlichen Antikörpermimetikum, wie z.B. einem molekulargeprägten Polymer (MIP), wobei das molekulargeprägte Polymer (MIP) vorzugsweise ein Acrylamid-basiertes Polymer, ein Polyethylen-basiertes Polymer, ein Acrylsäurebasiertes Polymer oder ein Derivat davon, wie z.B. ein Methacrylsäure-basiertes Polymer oder ein Methylmethacrylat-basiertes Polymer, oder jede Kombination oder Mischung davon ist, oder
(ii) einem Aptamer, noch bevorzugter einem Nucleinsäureaptamer oder einem Peptidaptamer, oder
(iii) Ribosomenbindungsprotein p34 (LRRC59) oder ER-Membrantranslokator SEC61alpha oder einem Fragment davon, ist.

13. Kit zum Identifizieren eines RNA-Moleküls oder mehrerer RNA-Moleküle in einem zellulären Extrakt, welche/s direkt oder indirekt assoziiert ist/sind mit wenigstens einem Ribosom, umfassend:
(i) ein Molekül, welches spezifisch an ein ribosomales Protein oder eine Gruppe von ribosomalen Proteinen bindend ist, wobei das Molekül an einem Substrat, einer Matrix oder einer Perle immobilisiert ist, und
(ii) Mittel zum Identifizieren eines RNA-Moleküls oder mehrerer RNA-Moleküle, welche/s direkt oder indirekt assoziiert ist/sind mit wenigstens einem Ribosom, umfassend das ribosomale Protein oder die Gruppe ribosomaler Proteine, wobei das RNA-Molekül vorzugsweise ein mRNA- oder nicht-codierendes RNA-Molekül, welches direkt assoziiert ist mit wenigstens einem Ribosom, oder ein nicht-codierendes Molekül, welches indirekt assoziiert ist mit wenigstens einem Ribosom, ist, und wobei das nicht-codierende RNA-Molekül vorzugsweise ein Mikro-RNA-Molekül, ein langes ncRNA-Molekül, ein kurzes ncRNA-Molekül, ein siRNA-Molekül, ein Antisense-RNA-Molekül oder ein regulatorisches RNA-Molekül ist.

14. Kit gemäß Anspruch 13, wobei das spezifisch bindende Molekül ein Nicht-Immunglobulin-Protein ist, vorzugsweise ausgewählt aus den Gruppen aus:
(i) Designed-Ankyrin-Repeat-Protein (DARPin), Affibody-Molekül, Adnectin, Anticalin, Affilin, Avimer, Knottin, Fynomer, Phylomer, Kunitz-Domänenpeptid und einem künstlichen Antikörpermimetikum, wie z.B. einem molekulargeprägten Polymer (MIP), wobei das molekulargeprägte Polymer (MIP) vorzugsweise ein Acrylamid-basiertes Polymer, ein Polyethylen-basiertes Polymer, ein Acrylsäurebasiertes Polymer oder ein Derivat davon, wie z.B. ein Methacrylsäure-basiertes Polymer oder ein Methylmethacrylat-basiertes Polymer, oder jede Kombination oder Mischung davon ist, oder
(ii) einem Aptamer, noch bevorzugter einem Nucleinsäureaptamer oder einem Peptidaptamer, oder
(iii) Ribosomenbindungsprotein p34 (LRRC59) oder ER-Membrantranslokator SEC61alpha oder einem Fragment davon.

15. Kit gemäß Anspruch 13 oder 14, wobei das ribosomale Protein ein ribosomales Säugetierprotein, vorzugsweise wenigstens ein ribosomales Protein, ausgewählt aus der Gruppe, bestehend aus: ribosomalem Homo sapiens Protein L3, ribosomalem Homo sapiens Protein L4, ribosomalem Homo sapiens Protein L5, ribosomalem Homo sapiens Protein L6, ribosomalem Homo sapiens Protein L7, ribosomalem Homo sapiens Protein L7A, ribosomalem Homo sapiens Protein L9, ribosomalem Homo sapiens Protein L10, ribosomalem Homo sapiens Protein L11, ribosomalem Homo sapiens Protein L12, ribosomalem Homo sapiens Protein L13, ribosomalem Homo sapiens Protein L13A, ribosomalem Homo sapiens Protein L14, ribosomalem Homo sapiens Protein L15, ribosomalem Homo sapiens Protein L17, ribosomalem Homo sapiens Protein L18, ribosomalem Homo sapiens Protein L18A, ribosomalem Homo sapiens Protein L19, ribosomalem Homo sapiens Protein L22, ribosomalem Homo sapiens Protein L23A, ribosomalem Homo sapiens Protein L26, ribosomalem Homo sapiens Protein L27A, ribosomalem Homo sapiens Protein L28, ribosomalem Homo sapiens Protein L29, ribosomalem Homo sapiens Protein L31, ribosomalem Homo sapiens Protein L32, ribosomalem Homo sapiens Protein L35, ribosomalem Homo sapiens Protein L35A ribosomalem Homo sapiens Protein L37 und ribosomalem Homo sapiens Protein L38, oder einem Homolog davon, oder aus einer eukaryotischen, prokaryotischen oder Archaenspezies, ist.

## Revendications

1. Procédé d'identification d'une ou plusieurs molécules d'ARN qui sont directement ou indirectement associées à au moins un ribosome, comprenant :
a) la mise en contact d'un extrait cellulaire avec une molécule se liant spécifiquement à une protéine ribosomique ou à un groupe de protéines ribosomiques, dans lequel ladite molécule se liant spécifiquement est immobilisée sur un substrat, une matrice ou une bille ; et
b) l'identification d'une ou plusieurs molécules d'ARN, qui sont directement ou indirectement associées à au moins un ribosome comprenant ladite protéine ribosomique ou ledit groupe de protéines ribosomiques.

2. Procédé selon la revendication 1, dans lequel ladite molécule d'ARN est une molécule d'ARNm ou d'ARN non codant, qui est directement associée à au moins un ribosome ; ou une molécule d'ARN non codant, qui est indirectement associée à au moins un ribosome, dans lequel ladite molécule d'ARN non codant est de préférence une molécule de micro-ARN, une molécule d'ARNnc long, une molécule d'ARNnc court, une molécule d'ARNsi, une molécule d'ARN antisens ou une molécule d'ARN régulateur.

3. Procédé selon la revendication 2, dans lequel la quasi-totalité ou la totalité des molécules d'ARNm ou d'ARN non codant qui sont directement associées à au moins un ribosome sont identifiées, ou dans lequel la quasi-totalité ou la totalité des molécules d'ARN non codant qui sont indirectement associées à au moins un ribosome sont identifiées, ou dans lequel ladite association indirecte avec au moins un ribosome comprend une association directe de ladite molécule d'ARN non codant avec ladite molécule d'ARNm, dans lequel ladite molécule d'ARNm est directement associée à au moins un ribosome.

4. Procédé selon l'une quelconque des revendications 1 à 3, dans lequel ladite identification d'une ou plusieurs molécules d'ARN comprend une amplification de la molécule d'ARN en une molécule d'acide nucléique, dans lequel ladite identification d'une ou plusieurs molécules d'ARN comprend de préférence une détermination de la séquence de la molécule d'ARN, de préférence par séquençage de l'ADN complémentaire soumis à une transcription inverse ou séquençage direct de l'ARN.

5. Procédé selon l'une quelconque des revendications 1 à 4, dans lequel ladite molécule se liant spécifiquement est un anticorps, un anticorps intracellulaire, une structure d'anticorps ou un anticorps modifié, ou un fragment de ceux-ci, ou une protéine autre qu'une immunoglobuline, choisie de préférence dans les groupes suivants :
i) une protéine de répétition ankyrine conçue (DARPine), une molécule d'afficorps, une adnectine, une anticaline, une affiline, un avimère, une knottine, un fynomère, un phylomère, un peptide du domaine de Kunitz et un anticorps mimétique artificiel tel qu'un polymère à empreinte moléculaire (MIP), dans lequel ledit polymère à empreinte moléculaire (MIP) est de préférence un polymère à base d'acrylamide, un polymère à base de polyéthylène, un polymère à base d'acide acrylique ou un dérivé de ceux-ci tel qu'un polymère à base d'acide méthacrylique ou un polymère à base de méthacrylate de méthyle, ou une combinaison ou un mélange quelconque de ceux-ci ; ou
ii) un aptamère, de préférence un aptamère d'acide nucléique ou un aptamère de peptide ; ou
iii) une protéine p34 de liaison au ribosome (LRRC59) ou un agent de translocation membranaire ER SEC61alpha, ou un fragment de ceux-ci.

6. Procédé selon l'une quelconque des revendications 1 à 5, dans lequel ladite protéine ribosomique est une protéine ribosomique eucaryote ou un homologue procaryote ou archéen de celle-ci, dans lequel ladite protéine ribosomique eucaryote est de préférence une protéine ribosomique de mammifère, de préférence encore au moins une protéine ribosomique choisie dans le groupe suivant : la protéine ribosomique L3 d'Homo sapiens, la protéine ribosomique L4 d'Homo sapiens, la protéine ribosomique L5 d'Homo sapiens, la protéine ribosomique L6 d'Homo sapiens, la protéine ribosomique L7 d'Homo sapiens, la protéine ribosomique L7A d'Homo sapiens, la protéine ribosomique L9 d'Homo sapiens, la protéine ribosomique L10 d'Homo sapiens, la protéine ribosomique d'Homo sapiens L11, la protéine ribosomique L12 d'Homo sapiens, la protéine ribosomique L13 d'Homo sapiens, la protéine ribosomique L13A d'Homo sapiens, la protéine ribosomique L14 d'Homo sapiens, la protéine ribosomique L15 d'Homo sapiens, la protéine ribosomique L17 d'Homo sapiens, la protéine ribosomique L18 d'Homo sapiens, la protéine ribosomique L18A d'Homo sapiens, la protéine ribosomique L19 d'Homo sapiens, la protéine ribosomique L22 d'Homo sapiens, la protéine ribosomique L23A d'Homo sapiens, la protéine ribosomique L26 d'Homo sapiens, la protéine ribosomique L27A d'Homo sapiens, la protéine ribosomique L28 d'Homo sapiens, la protéine ribosomique L29 d'Homo sapiens, la protéine ribosomique L31 d'Homo sapiens, la protéine ribosomique L32 d'Homo sapiens, la protéine ribosomique L35 d'Homo sapiens, la protéine ribosomique L35A d'Homo sapiens, la protéine ribosomique L37 d'Homo sapiens et la protéine ribosomique L38 d'Homo sapiens, ou un homologue de celles-ci d'une espèce eucaryote, procaryote ou archéenne.

7. Procédé selon l'une quelconque des revendications 1 à 6, dans lequel ledit extrait cellulaire est un extrait dérivé d'une cellule procaryote, d'une cellule archéobactérienne, d'une cellule eucaryote, d'une mitochondrie ou du cytoplasme d'une cellule eucaryote, dans lequel ladite mitochondrie est de préférence une mitochondrie d'une cellule de mammifère, de manière plus préférée une mitochondrie d'une cellule humaine, ou dans lequel ledit extrait cellulaire est un extrait de tissu dérivé d'un organisme eucaryote, de préférence un extrait de tissu mammifère, de manière plus préférée un extrait de tissu humain.

8. Procédé selon l'une quelconque des revendications 1 à 7, dans lequel ledit extrait cellulaire ou tissulaire est dérivé de cellules ou de tissus cultivés ou obtenus dans des conditions choisies dans le groupe suivant : des conditions de stress métabolique, des conditions spécifiques de nutriment et/ou d'apport, des conditions liées à l'absence ou à la présence d'un ou plusieurs facteurs de croissance, facteurs de différenciation ou ligands de récepteur, des conditions liées à l'absence ou à la présence de composés pharmaceutiquement actifs, de composés potentiellement actifs pharmaceutiquement ou de petites molécules, des conditions toxiques ou potentiellement toxiques, des conditions pathologiques ou potentiellement pathologiques ; et des conditions normales, saines ou optimales, dans lequel ledit procédé comprend de préférence l'identification d'une ou plusieurs molécules d'ARN associées à au moins un ribosome dans deux conditions ou plus choisies dans le groupe suivant : des conditions de stress métabolique, des conditions spécifiques de nutriment et/ou d'apport, des conditions liées à l'absence ou à la présence d'un ou plusieurs facteurs de croissance, facteurs de différenciation ou ligands de récepteur, des conditions liées à l'absence ou à la présence de composés pharmaceutiquement actifs, de composés potentiellement actifs pharmaceutiquement ou de petites molécules, des conditions toxiques ou potentiellement toxiques et/ou des conditions pathologiques ou potentiellement pathologiques et des conditions normales, saines ou optimales, comportant de préférence une comparaison desdites molécules d'ARN identifiées obtenues dans deux ou plus desdites conditions.

9. Procédé d'identification d'une ou plusieurs molécules d'ARN qui sont associées différemment à au moins un ribosome, comprenant :
a) la mise en contact d'un extrait cellulaire, dans lequel ledit extrait cellulaire est dérivé de cellules cultivées ou obtenues dans des conditions choisies dans le groupe suivant : des conditions de stress métabolique, différentes conditions de nutriment et/ou d'apport, des conditions liées à l'absence ou à la présence d'un ou plusieurs facteurs de croissance, facteurs de différenciation, ligands de récepteur, des conditions liées à l'absence ou à la présence de composés pharmaceutiquement actifs, de composés potentiellement actifs pharmaceutiquement ou de petites molécules, des conditions toxiques ou potentiellement toxiques et/ou des conditions pathologiques ou potentiellement pathologiques, avec une molécule se liant spécifiquement à une protéine ribosomique ou à un groupe de protéines ribosomiques, dans lequel ladite molécule se liant spécifiquement est immobilisée sur un substrat, une matrice ou une bille ;
b) la mise en contact d'un extrait cellulaire, dans lequel ledit extrait cellulaire est dérivé de cellules cultivées dans des conditions normales, saines et/ou optimales, avec une molécule se liant spécifiquement à une protéine ribosomique ou à un groupe de protéines ribosomiques, dans lequel ladite molécule se liant spécifiquement est immobilisée sur un substrat, une matrice ou une bille ; et
c) l'identification d'une ou plusieurs molécules d'ARN qui sont directement ou indirectement associées à au moins un ribosome comprenant ladite protéine ribosomique ou ledit groupe de protéines ribosomiques et qui sont présentes dans les conditions de l'étape a), mais pas dans les conditions de l'étape b), ou d'une ou plusieurs molécules d'ARN qui sont présentes dans les conditions de l'étape b), mais pas dans les conditions de l'étape a).

10. Procédé selon la revendication 9, dans lequel ladite molécule d'ARN est une molécule d'ARNm ou d'ARN non codant qui est directement associée à au moins un ribosome ou une molécule d'ARN non codant, qui est indirectement associée à au moins un ribosome, dans lequel ladite molécule d'ARN non codant est de préférence une molécule de micro-ARN, une molécule d'ARNnc long, une molécule d'ARNnc court, une molécule d'ARNsi, une molécule d'ARN antisens ou une molécule d'ARN régulateur.

11. Procédé selon les revendications 9 ou 10, dans lequel ladite identification d'une ou plusieurs molécules d'ARN comprend une amplification de la molécule d'ARN en une molécule d'acide nucléique ou une détermination de la séquence de la molécule d'ARN, de préférence par séquençage de l'ADN complémentaire soumis à une transcription inverse ou séquençage direct de l'ARN.

12. Procédé selon l'une quelconque des revendications 9 à 11, dans lequel ladite molécule se liant spécifiquement est un anticorps, un anticorps intracellulaire, une structure d'anticorps ou un anticorps modifié, ou un fragment de ceux-ci, ou une protéine autre qu'une immunoglobuline, dans lequel ladite protéine autre qu'une immunoglobuline est choisie de préférence dans les groupes suivants :
i) une protéine de répétition ankyrine conçue (DARPine), une molécule d'afficorps, une adnectine, une anticaline, une affiline, un avimère, une knottine, un fynomère, un phylomère, un peptide du domaine de Kunitz et un anticorps mimétique artificiel tel qu'un polymère à empreinte moléculaire (MIP), dans lequel ledit polymère à empreinte moléculaire (MIP) est de préférence un polymère à base d'acrylamide, un polymère à base de polyéthylène, un polymère à base d'acide acrylique ou un dérivé de ceux-ci-ci tel qu'un polymère à base d'acide méthacrylique ou un polymère à base de méthacrylate de méthyle, ou une combinaison ou un mélange quelconque de ceux-ci ; ou
ii) un aptamère, de préférence un aptamère d'acide nucléique ou un aptamère de peptide ; ou
iii) une protéine p34 de liaison au ribosome (LRRC59) ou un agent de translocation membranaire ER SEC61alpha, ou un fragment de ceux-ci.

13. Kit permettant d'identifier, dans un extrait cellulaire, une ou plusieurs molécules d'ARN qui sont directement ou indirectement associées à au moins un ribosome, comprenant :
(i) une molécule qui se lie spécifiquement à une protéine ribosomique ou à un groupe de protéines ribosomiques, dans lequel ladite molécule est immobilisée sur un substrat, une matrice ou une bille ; et
(ii) des moyens permettant d'identifier une ou plusieurs molécules d'ARN, qui sont directement ou indirectement associées à au moins un ribosome comprenant ladite protéine ribosomique ou ledit groupe de protéines ribosomiques, dans lequel ladite molécule d'ARN est de préférence une molécule d'ARNm ou d'ARN non codant qui est directement associée à au moins un ribosome ou une molécule d'ARN non codant, qui est indirectement associée à au moins un ribosome, et dans lequel ladite molécule d'ARN non codant est de préférence une molécule de micro-ARN, une molécule d'ARNnc long, une molécule d'ARNnc court, une molécule d'ARNsi, une molécule d'ARN antisens ou une molécule d'ARN régulateur.

14. Kit selon la revendication 13, dans lequel ladite molécule se liant spécifiquement est une protéine autre qu'une immunoglobuline, choisie de préférence dans les groupes suivants :
i) une protéine de répétition ankyrine conçue (DARPine), une molécule d'afficorps, une adnectine, une anticaline, une affiline, un avimère, une knottine, un fynomère, un phylomère, un peptide du domaine de Kunitz et un anticorps mimétique artificiel tel qu'un polymère à empreinte moléculaire (MIP), dans lequel ledit polymère à empreinte moléculaire (MIP) est de préférence un polymère à base d'acrylamide, un polymère à base de polyéthylène, un polymère à base d'acide acrylique ou un dérivé de ceux-ci tel qu'un polymère à base d'acide méthacrylique ou un polymère à base de méthacrylate de méthyle, ou une combinaison ou un mélange quelconque de ceux-ci ; ou
ii) un aptamère, de préférence un aptamère d'acide nucléique ou un aptamère de peptide ; ou
iii) une protéine p34 de liaison au ribosome (LRRC59) ou un agent de translocation membranaire ER SEC61alpha, ou un fragment de ceux-ci.

15. Kit selon les revendications 13 ou 14, dans lequel ladite protéine ribosomique est une protéine ribosomique de mammifère, de préférence au moins une protéine ribosomique choisie dans le groupe suivant : la protéine ribosomique L3 d'Homo sapiens, la protéine ribosomique L4 d'Homo sapiens, la protéine ribosomique L5 d'Homo sapiens, la protéine ribosomique L6 d'Homo sapiens, la protéine ribosomique L7 d'Homo sapiens, la protéine ribosomique L7A d'Homo sapiens, la protéine ribosomique L9 d'Homo sapiens, la protéine ribosomique L10 d'Homo sapiens, la protéine ribosomique d'Homo sapiens L11, la protéine ribosomique L12 d'Homo sapiens, la protéine ribosomique L13 d'Homo sapiens, la protéine ribosomique L13A d'Homo sapiens, la protéine ribosomique L14 d'Homo sapiens, la protéine ribosomique L15 d'Homo sapiens, la protéine ribosomique L17 d'Homo sapiens, la protéine ribosomique L18 d'Homo sapiens, la protéine ribosomique L18A d'Homo sapiens, la protéine ribosomique L19 d'Homo sapiens, la protéine ribosomique L22 d'Homo sapiens, la protéine ribosomique L23A d'Homo sapiens, la protéine ribosomique L26 d'Homo sapiens, la protéine ribosomique L27A d'Homo sapiens, la protéine ribosomique L28 d'Homo sapiens, la protéine ribosomique L29 d'Homo sapiens, la protéine ribosomique L31 d'Homo sapiens, la protéine ribosomique L32 d'Homo sapiens, la protéine ribosomique L35 d'Homo sapiens, la protéine ribosomique L35A d'Homo sapiens, la protéine ribosomique L37 d'Homo sapiens et la protéine ribosomique L38 d'Homo sapiens, ou un homologue de celles-ci d'une espèce eucaryote, procaryote ou archéenne.


### SEQUENCE LISTING

<110> University of Surrey
<120> MEANS AND METHODS FOR IDENTIFYING RIBOSOME ASSOCIATED RNA MOLECULES
<130> N 8663 / OL
<160> 8
<170> PatentIn version 3.5
<210> 1
   <211> 20
   <212> DNA
   <213> Artificial Sequence
<220>
   <223> Primer p53 Fwd
<400> 1
   actgggacgg aacagctttg 20
<210> 2
   <211> 20
   <212> DNA
   <213> Artificial Sequence
<220>
   <223> Primer p53 Rev
<400> 2
   agaggagctg gtgttgttgg 20
<210> 3
   <211> 20
   <212> DNA
   <213> Artificial Sequence
<220>
   <223> Primer NFkB-p105 Fwd
<400> 3
   tgcactgtaa ctgctggacc 20
<210> 4
   <211> 20
   <212> DNA
   <213> Artificial Sequence
<220>
   <223> Primer NFkB-p105 Rev
<400> 4
   aataggcaag gtcagggtgc 20
<210> 5
   <211> 20
   <212> DNA
   <213> Artificial Sequence
<220>
   <223> Primer GAPDH Fwd
<400> 5
   ggctctccag aacatcatcc 20
<210> 6
   <211> 20
   <212> DNA
   <213> Artificial Sequence
<220>
   <223> Primer GAPDH Rev
<400> 6
   tttctagacg gcaggtcagg 20
<210> 7
   <211> 20
   <212> DNA
   <213> Artificial Sequence
<220>
   <223> Primer RNU6 Fw
<400> 7
   cgcttcggca gcacatatac 20
<210> 8
   <211> 20
   <212> DNA
   <213> Artificial Sequence
<220>
   <223> Primer RNU6 Rev
<400> 8
   ggaacgcttc acgaatttgc 20